# EUROPEAN PATENT APPLICATION

(11) **EP 4 269 411 A1**
(43) Date of publication of application: **01.11.2023**
(21) Application number: 21910779.4
(22) Date of filing: 21.12.2021
(51) Int. Cl.: C07D 471/22, C07F 15/02, C07F 15/06, H01M 12/06, H01M 12/08, H01M 4/88, H01M 4/90, B01J 37/04, B01J 37/08, C07D 487/22, H01M 8/10, H01M 8/12, B01J 31/22

(54) **METAL COMPLEX OR ADDUCT THEREOF, CATALYST COMPRISING METAL COMPLEX OR ADDUCT THEREOF AND METHOD FOR PRODUCING SAME, LIQUID COMPOSITION OR ELECTRODE CONTAINING CATALYST, AND AIR BATTERY OR FUEL CELL EQUIPPED WITH ELECTRODE**

(30) Priority: 24.12.2020 JP 2020215457
(71) Applicant: Azul Energy Inc., Sendai-shi, Miyagi 980-0811 (JP)
(72) Inventor: ITO Koju, Sendai-shi, Miyagi 980-0811 (JP); NAKAMURA Koki, Sendai-shi, Miyagi 980-0811 (JP)
(74) Representative: Müller, Christian Stefan Gerd
(86) International application number: PCT/JP2021/047311
(87) International publication number: WO 2022/138636

(57) **Abstract**

To provide a metal complex or adduct thereof having excellent oxygen reduction catalyst performance, a catalyst including the metal complex or adduct thereof and a method of producing the catalyst, a liquid composition or electrode including the catalyst, and an air battery or fuel cell including the electrode.

A metal complex or adduct thereof represented by Formula (1) or (2), a catalyst including the metal complex or adduct thereof, a method of producing the catalyst, a liquid composition or electrode including the catalyst, and an air battery or fuel cell including the electrode.

## Description

### [Technical Field]

The present invention relates to a metal complex or adduct thereof, a catalyst including the metal complex or adduct thereof, a method of producing the catalyst, a liquid composition or electrode including the catalyst, and an air battery or fuel cell including the electrode.

### [Background Art]

Fuel cells and air batteries are known as devices that convert chemical energy into electrical energy using an oxidation-reduction reaction in electrodes. In air electrodes of these batteries, a reduction reaction of oxygen occurs, and a catalyst is used to promote the reduction reaction. As representative catalysts, in the case of fuel cells, carbon materials that support platinum are known, and in the case of air batteries, carbon materials that support manganese dioxide are known.

However, since rare metals such as platinum are expensive and resource amounts thereof are limited, there have been attempts to develop catalysts using materials that are cheaper and more abundantly available. For example, Patent Document 1 discloses an air electrode catalyst containing iron phthalocyanine and a carbon material as a co-catalyst. However, since iron phthalocyanine is less likely to adsorb to the surface of the carbon material, its oxygen reduction performance is not sufficient.

On the other hand, Patent Document 2 discloses use of graphene oxide as a carbon material that is a co-catalyst in combination with iron phthalocyanine. However, in the method of producing an oxygen reduction catalyst disclosed in Patent Document 2, since it is necessary to form a complex of iron phthalocyanine and graphene oxide temporarily, and then reduce the graphene oxide to obtain a complex of the iron phthalocyanine and graphene, there is a problem of a complicated production process.

Patent Document 3 discloses an oxidation/reduction electrode containing a cobalt tetrapyrazinoporphyrazine derivative represented by a specific structural formula as a catalyst component. However, in the cobalt tetrapyrazinoporphyrazine derivative described in Patent Document 3, since a trifluoromethyl group is bonded to pyrazine, there is a risk of oxygen reduction catalyst performance deteriorating.

### [Citation List]

### [Patent Document]

[Patent Document 1]
   Japanese Unexamined Patent Application, First Publication No. 2016-85925
[Patent Document 2]
   Japanese Unexamined Patent Application, First Publication No. 2014-91061
[Patent Document 3]
   PCT International Publication No. WO2007/023964

### [Summary of Invention]

### [Technical Problem]

Therefore, it has been desired to develop a catalyst material and a catalyst having excellent oxygen reduction catalyst performance without using rare metals such as platinum. In addition, there is a demand for a method of producing a catalyst that can be easily adsorbed to a conductive material as a co-catalyst in a simple process.

The present invention has been made to address the above problems in the related art, and an object of the present invention is to provide a metal complex or adduct thereof having excellent oxygen reduction catalyst performance, a catalyst including the metal complex or adduct thereof and a method of producing the catalyst, a liquid composition or electrode including the catalyst, and an air battery or fuel cell including the electrode.

### [Solution to Problem]

The inventors have conducted extensive studies regarding the above problems, and as a result, found that, unexpectedly, a metal complex or adduct thereof having a specific chemical structure has excellent oxygen reduction catalyst performance, and completed the present invention.

An object of the present invention is achieved by a metal complex or adduct thereof represented by the following Formula (1) or (2): 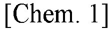 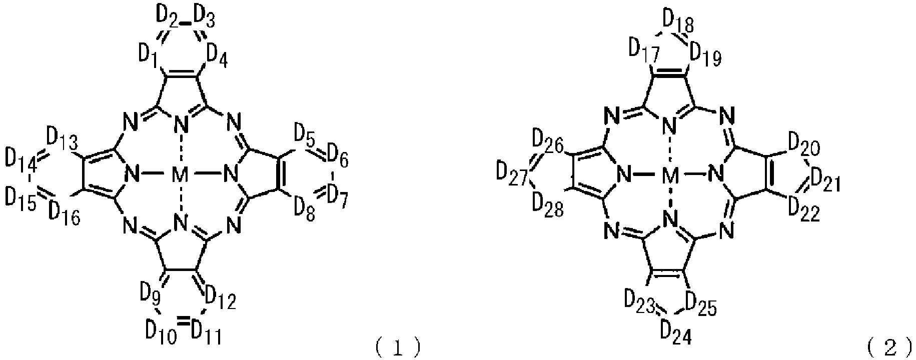 (where,
M represents an iron atom or a cobalt atom,
D¹ to D²⁸ each independently represent a nitrogen atom, a sulfur atom or a carbon atom, and when D¹ to D²⁸ are carbon atoms, the carbon atoms may be each independently bonded to a hydrogen atom, a halogen atom, an alkyl group, a cycloalkyl group, an alkenyl group, an alkynyl group, an aryl group, an alkylsulfonyl group, an alkoxy group or an alkylthio group)
where, in Formula (1), when the number of carbon atoms among D¹ to D¹⁶ is 8 or less, at least one of the carbon atoms is bonded to a halogen atom, an alkyl group, a cycloalkyl group, an alkenyl group, an alkynyl group, an aryl group, an alkylsulfonyl group, an alkoxy group or an alkylthio group or
the metal complex of Formula (1) forms an adduct.

D¹ to D¹⁶ are preferably a nitrogen atom or a carbon atom, and D¹⁷ to D²⁸ are preferably a sulfur atom or a carbon atom.

The metal complex or adduct thereof is preferably represented by the following Formulae:

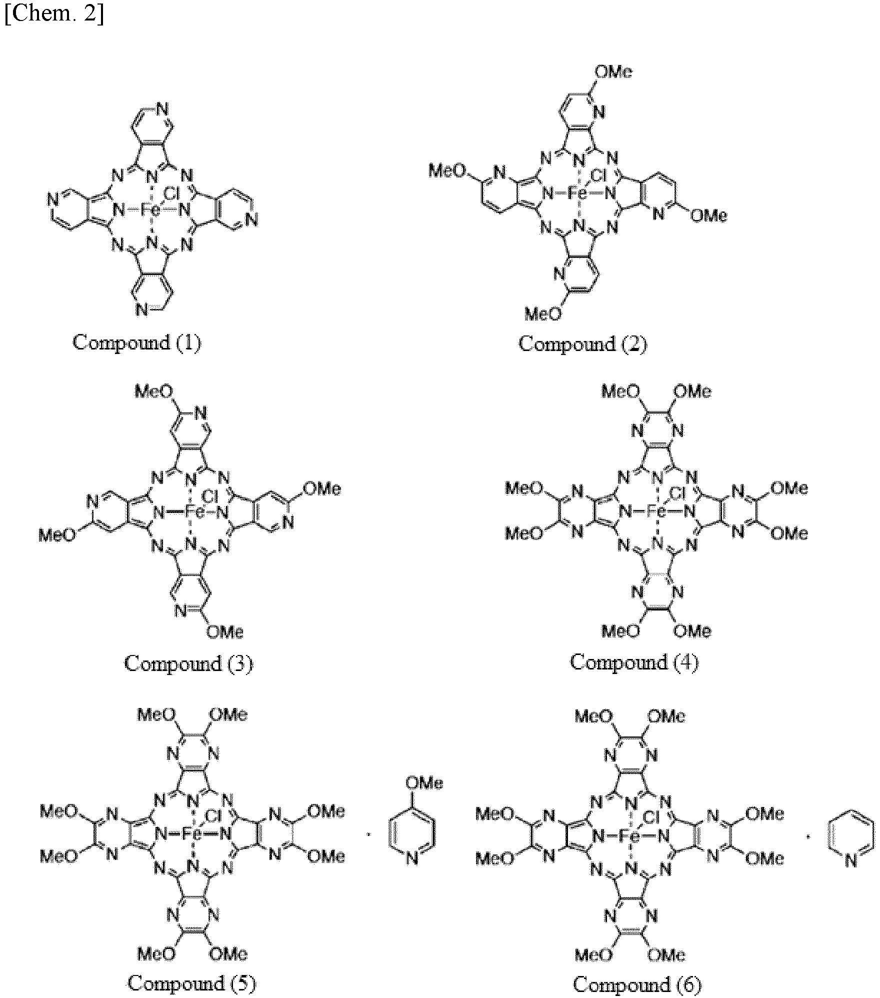

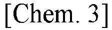

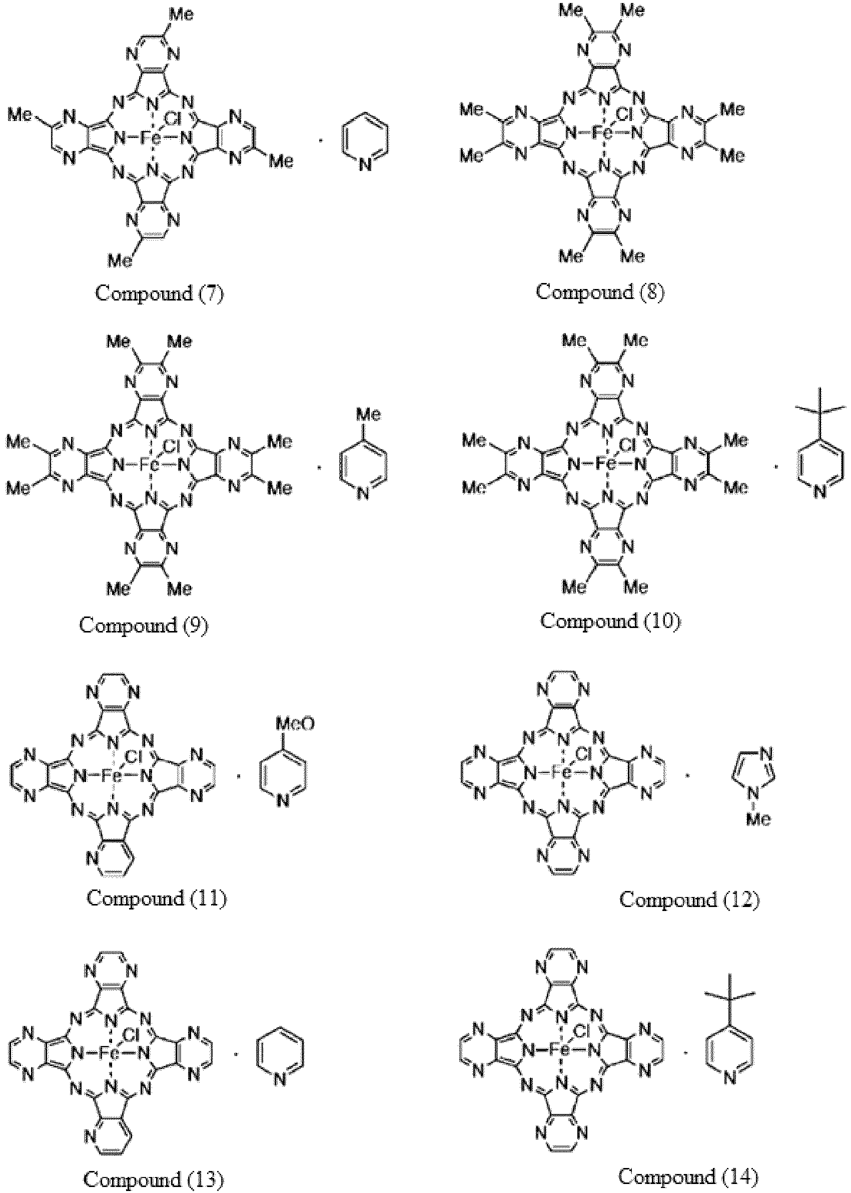

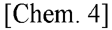

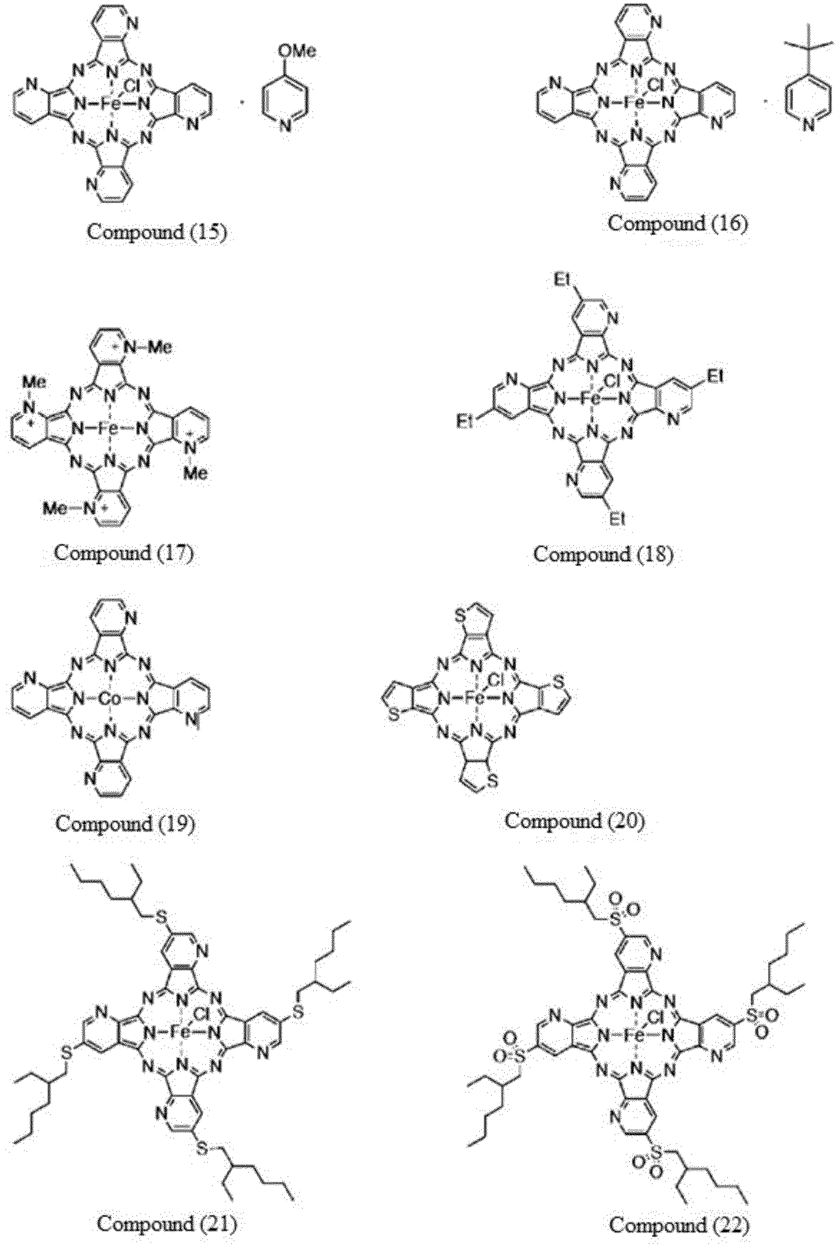

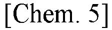

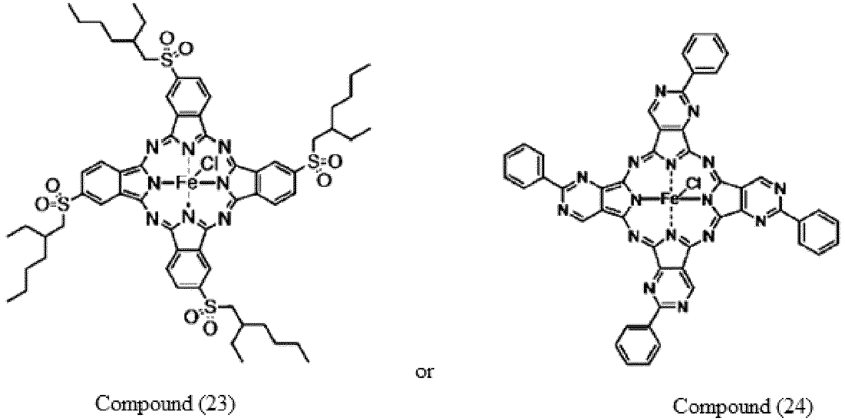

The present invention relates to a catalyst including the metal complex or adduct thereof of the present invention and a conductive material.

The content of the metal complex or adduct thereof is preferably 75 mass% or less with respect to a total amount of 100 mass% of the metal complex or adduct thereof and the conductive material.

The conductive material preferably contains a carboxyl group.

The content of the carboxyl group is preferably 20 mass% or less with respect to 100 mass% of the conductive material.

The catalyst of the present invention is preferably used for oxygen reduction.

The present invention relates to a liquid composition including the catalyst of the present invention and a solvent.

The present invention relates to an electrode including the catalyst of the present invention.

The present invention relates to an air battery or fuel cell including the electrode of the present invention.

In addition, the present invention relates to a method of producing the catalyst of the present invention, including:
(a) a step of dissolving the metal complex or adduct thereof in a solvent to prepare a solution;
(b) a step of dispersing the conductive material in the solution to prepare a dispersion; and
(c) a step of removing the solvent from the dispersion.

The steps (a) and (b) are preferably performed at a temperature equal to or lower than a boiling point of the solvent.

The solubility of the metal complex or adduct thereof with respect to the solvent is preferably 0.1 g/L or more.

### [Advantageous Effects of Invention]

According to the present invention, it is possible to provide a catalyst having excellent oxygen reduction catalyst performance using a metal complex or adduct thereof of the present invention. In addition, since the metal complex or adduct thereof of the present invention is easily adsorbed on a conductive material, it is possible to produce a catalyst without performing a complicated production process.

In addition, the present invention can provide a catalyst for an air battery or fuel cell at relatively low cost because it is possible to obtain excellent oxygen reduction catalyst performance without using rare metals such as platinum.

### [Brief Description of Drawings]

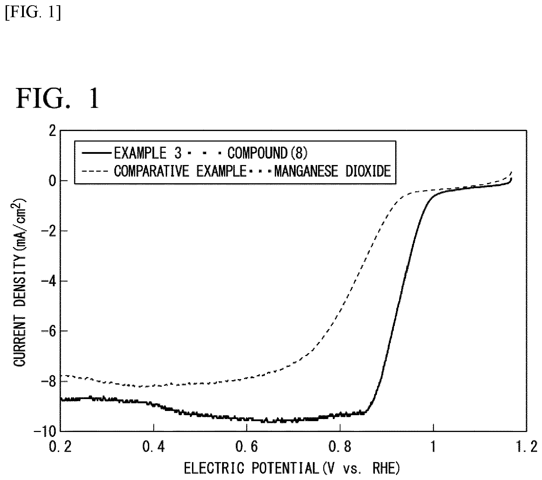

FIG. 1 shows examples of the results of LSV measurement by RRDE.

### [Description of Embodiments]

### [Metal complex or adduct thereof]

A metal complex or adduct thereof of the present invention is represented by the following Formula (1) or (2): 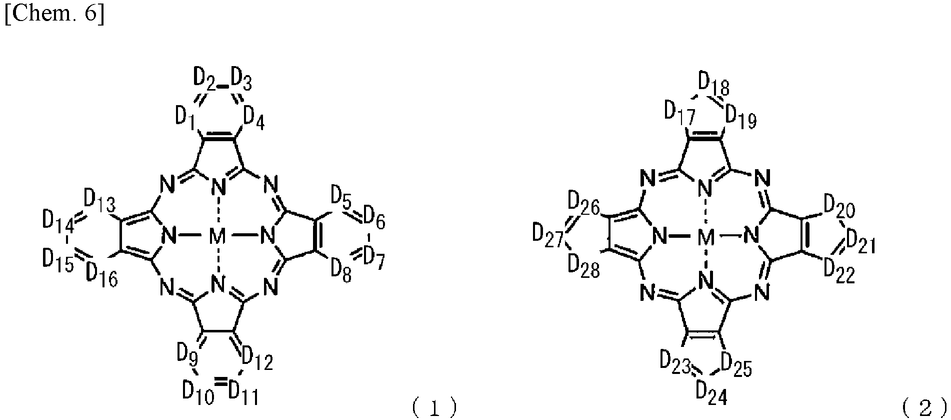 (where,
M represents an iron atom or a cobalt atom,
D¹ to D²⁸ each independently represent a nitrogen atom, a sulfur atom or a carbon atom, and when D¹ to D²⁸ are carbon atoms, the carbon atoms may be each independently bonded to a hydrogen atom, a halogen atom, an alkyl group, a cycloalkyl group, an alkenyl group, an alkynyl group, an aryl group, an alkylsulfonyl group, an alkoxy group or an alkylthio group)
where, in Formula (1), when the number of carbon atoms among D¹ to D¹⁶ is 8 or less, at least one of the carbon atoms is bonded to a halogen atom, an alkyl group, a cycloalkyl group, an alkenyl group, an alkynyl group, an aryl group, an alkylsulfonyl group, an alkoxy group or an alkylthio group or
the metal complex of Formula (1) forms an adduct.

The bond between a nitrogen atom and M means coordination of a nitrogen atom to M. A halogen atom, a hydroxyl group, or a hydrocarbon group having 1 to 8 carbon atoms may be additionally bonded as a ligand to M. In addition, an anionic counterion may be present so that the state becomes electrically neutral. In addition, an adduct to which an electrically neutral molecule is added may be present.

The valence of M is not particularly limited. A halogen atom, a hydroxyl group or an alkoxy group (alkyloxy group) having 1 to 8 carbon atoms may be bonded as a ligand (for example, an axial ligand) or an anionic counterion may be present so that the metal complex or adduct thereof is electrically neutral. Examples of anionic counterions include halide ions, hydroxide ions, nitrate ions, and sulfate ions. In addition, the structure of the alkyl group of the alkoxy group (alkyloxy group) having 1 to 8 carbon atoms may be linear, branched, or cyclic.

In the present invention, examples of halogen atoms include fluorine, chlorine, bromine, and iodine atoms.

In the present invention, the alkyl group represents a linear or branched monovalent hydrocarbon group. The number of carbon atoms in the alkyl group is preferably 1 to 20, more preferably 1 to 12, and still more preferably 1 to 6. Examples of alkyl groups include a methyl group, ethyl group, n-propyl group, isopropyl group, n-butyl group, isobutyl group, sec-butyl group, tert-butyl group, n-pentyl group, isopentyl group, sec-pentyl group, tert-pentyl group, and n-hexyl group.

In the present invention, the cycloalkyl group represents a cyclic monovalent hydrocarbon group. The number of carbon atoms in the cycloalkyl group is preferably 3 to 20, more preferably 3 to 12, and still more preferably 3 to 6. Examples of cycloalkyl groups include a cyclopropyl group, cyclobutyl group, cyclopentyl group, cyclohexyl group, 1-methylcyclopropyl group, 2-methylcyclopropyl group, and 2,2-dimethylcyclopropyl group.

In the present invention, the alkenyl group represents a linear or branched monovalent hydrocarbon group having a double bond. The number of carbon atoms in the alkenyl group is preferably 2 to 20, more preferably 2 to 12, and still more preferably 2 to 6. Examples of alkenyl groups include a vinyl group, 1-propenyl group, 2-propenyl group, 1-butenyl group, 2-butenyl group, 3-butenyl group, 1-methyl-2-propenyl group, 2-methyl-2-propenyl group, 1-pentenyl group, 2-pentenyl group, 3-pentenyl group, 4-pentenyl group, 1-methyl-2-butenyl group, 2-methyl-2-butenyl group, 1-hexenyl group, 2-hexenyl group, 3-hexenyl group, 4-hexenyl group, and 5-hexenyl group.

In the present invention, the alkynyl group represents a linear or branched monovalent hydrocarbon group having a triple bond. The number of carbon atoms in the alkynyl group is preferably 2 to 20, more preferably 2 to 12, and still more preferably 2 to 6. Examples of alkynyl groups include an ethynyl group, 1-propynyl group, 2-propynyl group, 1-butyn-1-yl group (1-butynyl group), 2-butyn-1-yl group (2-butynyl group), 3-butyn-1-yl group (3-butynyl group), 1-methyl-2-propyn-1-yl group (1-methyl-2-propynyl group), 2-methyl-3-butyn-2-yl group (2-methyl-3-butynyl group), 1-pentyn-1-yl group (1-pentynyl group), 2-pentyn-1-yl group (2-pentynyl group), 3-pentyn-2-yl group (3-pentynyl group), 4-pentyn-1-yl group (4-pentynyl group), 1-methyl-2-butynyl group (1-methyl-2-butyn-1-yl group), 2-methyl-3-pentyn-1-yl group (2-methyl-3-pentynyl group), 1-hexyne-1-yl group (1-hexynyl group), and 1,1-dimethyl-2-butyn-1-yl group (1,1-dimethyl-2-butynyl group).

In the present invention, the aryl group represents a monovalent aromatic hydrocarbon group. The number of carbon atoms in the aryl group is preferably 6 to 40, and more preferably 6 to 30. Examples of aryl groups include a phenyl group, biphenyl group, terphenyl group, naphthyl group, fluorenyl group, benzofluorenyl group, dibenzofluorenyl group, phenanthryl group, anthracenyl group, benzophenanthryl group, benzoanthracenyl group, chrysenyl group, pyrenyl group, fluoranthenyl group, triphenylenyl group, benzofluoranthenyl group, dibenzoanthracenyl group, perylenyl group, and helicenyl group.

In the present invention, the alkylsulfonyl group represents a monovalent group in which an alkyl group is bonded to a sulfonyl group. The alkyl group in the alkylsulfonyl group can be the group described as the above "alkyl group." The number of carbon atoms in the alkylsulfonyl group is preferably 1 to 20, more preferably 1 to 12, and still more preferably 1 to 6. Examples thereof include a methylsulfonyl group, ethyl sulfonyl group, n-propyl sulfonyl group, isopropyl sulfonyl group, n-butyl sulfonyl group, sec-butyl sulfonyl group, tert-butyl sulfonyl group, n-pentyl sulfonyl group, isopentyl sulfonyl group, tert-pentyl sulfonyl group, neopentyl sulfonyl group, 2,3-dimethylpropyl sulfonyl group, 1-ethylpropyl sulfonyl group, 1-methylbutyl sulfonyl group, n-hexyl sulfonyl group, isohexyl sulfonyl group, and 1,1,2-trimethylpropyl sulfonyl group.

In the present invention, the alkoxy group represents a monovalent group in which a hydrocarbon group is bonded via an ether bond. The number of carbon atoms in the alkoxy group is preferably 1 to 20, more preferably 1 to 12, and still more preferably 1 to 6. Examples of alkoxy groups include a methoxy group, ethoxy group, n-propoxy group, n-butoxy group, n-pentyloxy group, n-hexyloxy group, isopropoxy group, iso-butoxy group, sec-butoxy group, tert-butoxy group, and isohexyloxy group.

In the present invention, the alkylthio group represents a group in which an oxygen atom in an ether bond of an alkoxy group is substituted with a sulfur atom. The number of carbon atoms in the alkylthio group is preferably 1 to 20, more preferably 1 to 16, and still more preferably 1 to 12. Examples of alkylthio groups include a methylthio group, ethylthio group, n-propylthio group, n-butylthio group, n-pentylthio group, n-hexylthio group, and isopropylthio group.

An alkyl group, cycloalkyl group, alkenyl group, alkynyl group, aryl group, alkylsulfonyl group, alkoxy group, and alkylthio group may be unsubstituted substituents, or each may be substituted with one or more substituents such as a halogen, alkyl group, alkenyl group, alkynyl group, aryl group, alkoxy group, alkylthio group, cyano group, carbonyl group, carboxyl group, amino group, nitro group, silyl group, and sulfo group.

D¹ to D¹⁶ are preferably a nitrogen atom or a carbon atom, and D¹⁷ to D²⁸ are preferably a sulfur atom or a carbon atom. The number of nitrogen atoms among D¹ to D¹⁶ is preferably 2 to 12, and more preferably 4 to 8. The number of sulfur atoms among D¹⁷ to D²⁸ is preferably 2 to 10 and more preferably 4 to 8.

Preferably, the metal complex or adduct thereof of the present invention is a compound represented by the following formulae.

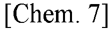

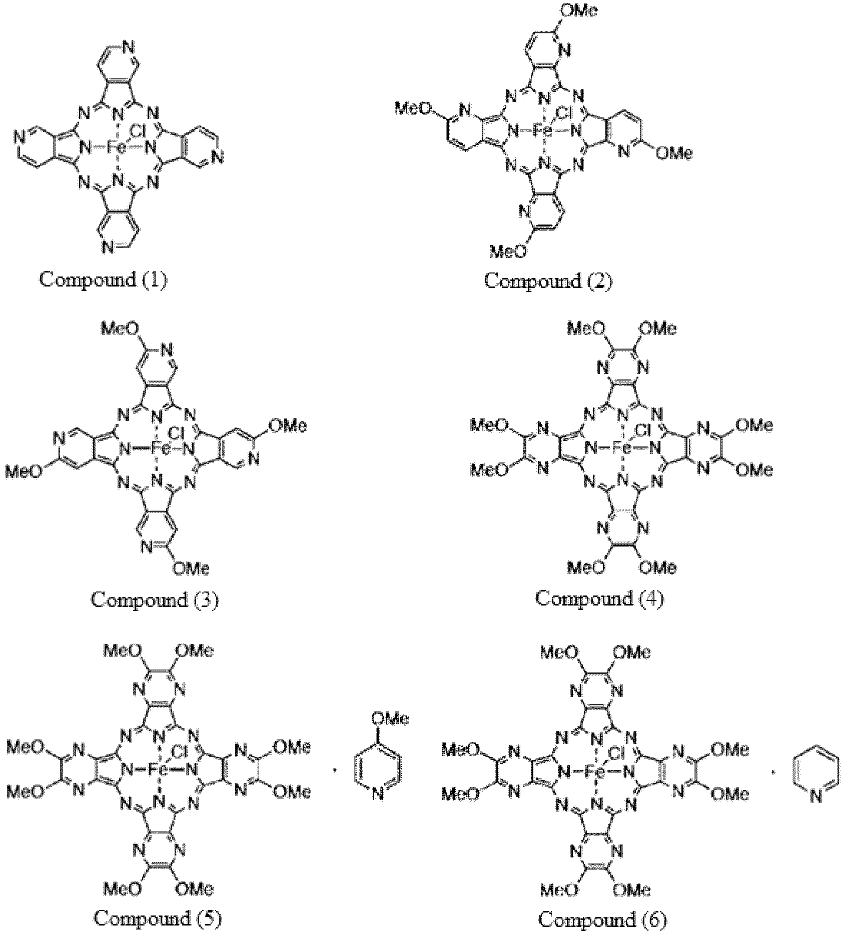

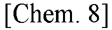

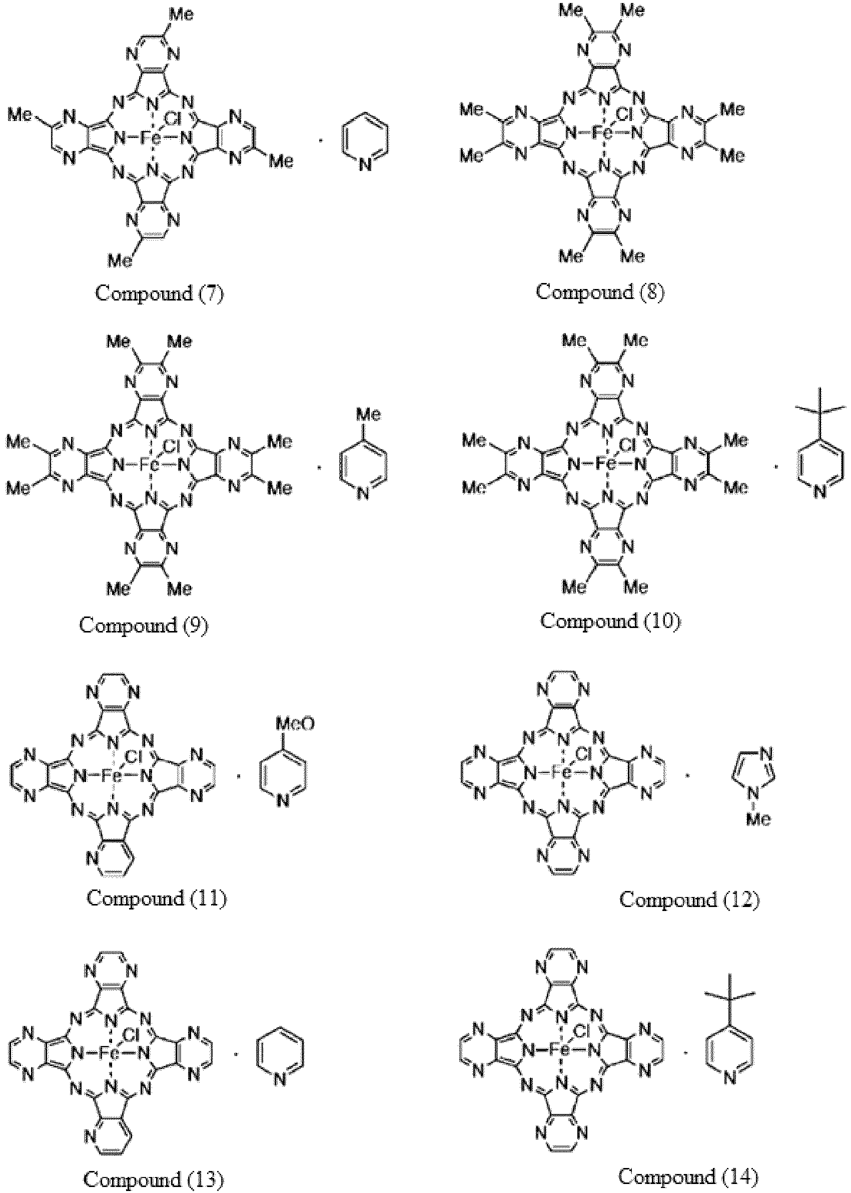

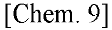

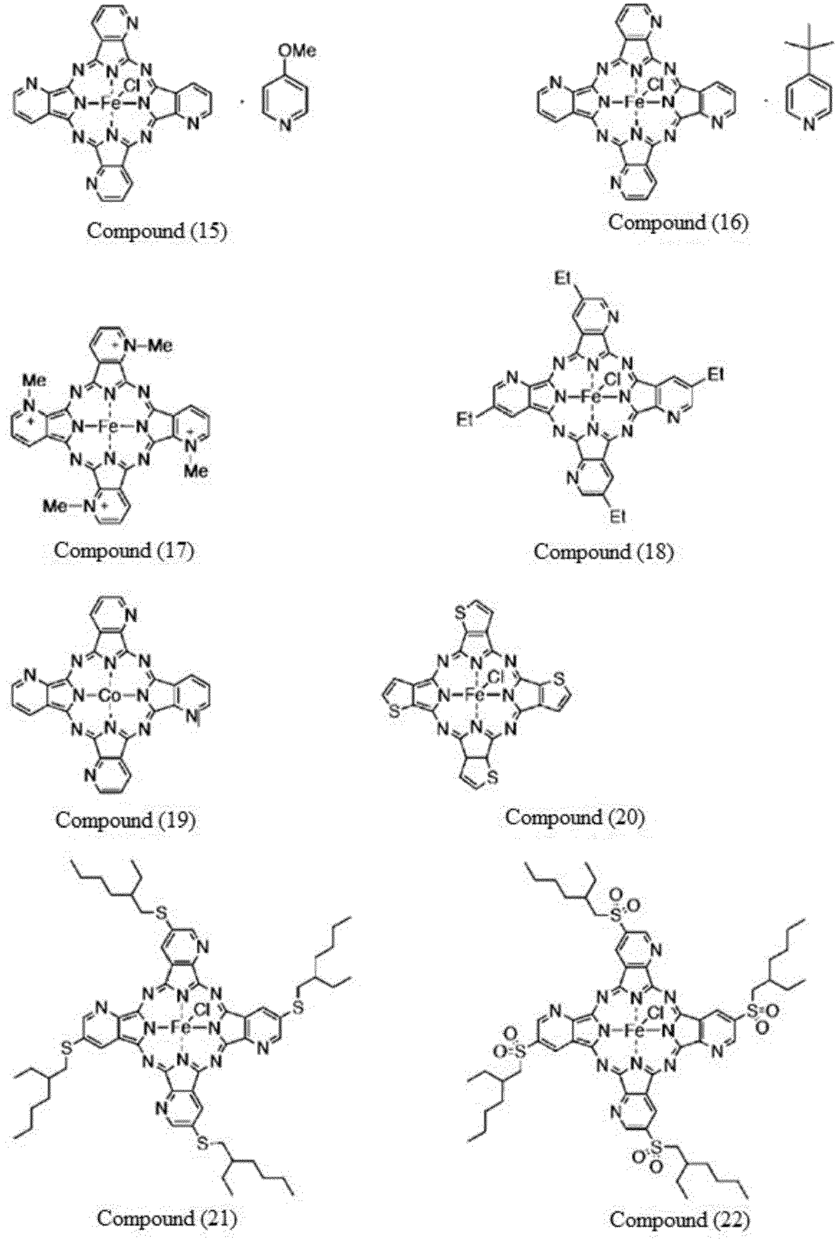

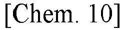

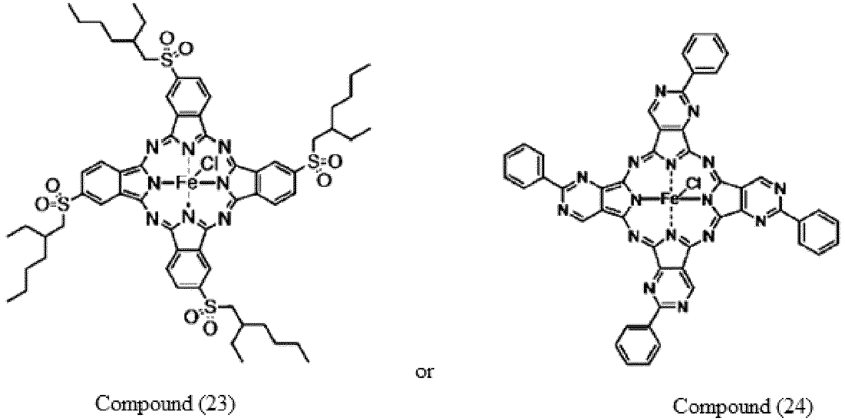

A method of producing a metal complex or adduct thereof is not particularly limited, and for example, a method of heating a dicyano compound such as pyridine-2,3-dicarbonitrile and a metal atom in an alcohol solvent in the presence of a basic substance may be exemplified. Here, examples of basic substances include inorganic bases such as potassium carbonate, sodium carbonate, calcium carbonate, sodium hydrogen carbonate, and sodium acetate; and organic bases such as triethylamine, tributylamine, and diazabicycloundecene.

### [Catalyst]

In one embodiment, the present invention relates to a catalyst including the metal complex or adduct thereof of the present invention and a conductive material. Only one type of metal complex or adduct thereof can be used or two or more types of metal complexes or adducts thereof can be used in combination.

The conductive material is not particularly limited as long as it has conductivity, and examples thereof include carbon materials, metal materials, and metal oxide materials. The carbon material is preferable as the conductive material. The conductive materials may be used alone or two or more thereof may be used in combination.

The carbon material is preferably derived from conductive carbon. Specific examples of carbon materials include graphite, amorphous carbon, activated carbon, graphene, carbon black, carbon fibers, mesocarbon microbeads, microcapsule carbon, fullerene, carbon nanoforms, carbon nanotubes, and carbon nanohorns. Among these, the carbon material is preferably graphite, amorphous carbon, activated carbon, graphene, carbon black, a carbon fiber, fullerene, or a carbon nanotube, and more preferably a carbon nanotube, carbon black, or graphene.

Examples of carbon nanotubes include single-walled carbon nanotubes (hereinafter referred to as "SWCNT"), double-walled carbon nanotubes (hereinafter referred to as "DWCNT"), and multi-walled carbon nanotubes (hereinafter referred to as "MWCNT").

The carbon material may have a heteroatom. Examples of heteroatoms include oxygen atoms, nitrogen atoms, phosphorus atoms, sulfur atoms, and silicon atoms. When the carbon material has a heteroatom, the carbon material may contain one type of heteroatom alone or may contain two or more types of heteroatoms. Here, the carbon material may be oxidized, hydroxylated, nitrided, phosphided, sulfided, or silicified.

Examples of metal materials include titanium and tin. In addition, examples of metal oxide materials include titanium oxide and tin oxide (SnO₂, ITO, ATO).

The conductive material may have a functional group such as a hydroxyl group, a carboxyl group, a nitrogen-containing group, a silicon-containing group, a phosphorus-containing group such as a phosphate group and a sulfur-containing group such as a sulfonic acid group. In particular, the carbon material preferably has a carboxyl group. When the conductive material has a carboxyl group, the metal complex or adduct thereof can be easily adsorbed on the surface of the conductive material, it is possible to improve the durability of the catalyst, and it is possible to further improve the oxygen reduction catalyst performance.

The conductive material may be subjected to a surface treatment according to an oxidation treatment. Particularly, when the carbon material such as carbon black is oxidized to impart a hydrophilic functional group such as a carboxyl group or a hydroxyl group, it is thus possible to improve the interaction with the metal complex, and it becomes possible to control the ionization potential to be within an optimal range. As an oxidation treatment method, known methods can be used, and a wet treatment of performing stirring and mixing in an oxidant aqueous solution such as nitric acid, sulfuric acid, or chloric acid, or a gas-phase treatment such as a plasma treatment or an ozone treatment can be used.

When the conductive material contains a carboxyl group, the content of the carboxyl group with respect to 100 mass% of the conductive material is preferably 20 mass% or less, more preferably 15 mass% or less, and still more preferably 10 mass% or less. When the content of the carboxyl group is equal to or less than the upper limit value, this is advantageous because the catalyst production cost is reduced. In addition, the content of the carboxyl group is preferably 1 mass% or more, more preferably 5 mass% or more, and still more preferably 8 mass% or more. When the content of the carboxyl group is equal to or more than the lower limit value, it is possible to further improve the durability of the catalyst and the oxygen reduction catalyst performance. Here, the content of the carboxyl group can be measured through elemental analysis, X-ray photoelectron spectroscopy or the like.

The specific surface area of the conductive material is preferably 0.8 m²/g or more, more preferably 10 m²/g or more, still more preferably 50 m²/g or more, particularly preferably 100 m²/g or more, and most preferably 500 m²/g or more. When the specific surface area is 0.8 m²/g or more, it is easy to increase the amount of the catalyst supported and it is possible to further improve the oxygen reduction catalyst performance of the catalyst. The upper limit value of the specific surface area is not particularly limited, and may be, for example, 2,000 m²/g. Here, the specific surface area can be measured by a specific surface area measuring device using a nitrogen adsorption BET method.

The average particle size of the conductive material is not particularly limited, and is, for example, preferably 5 nm to 1,000 µm, more preferably 10 nm to 100 µm, and still more preferably 50 nm to 10 µm. The following (A1) to (A3) are exemplified as methods of adjusting the average particle size of the conductive material to be within the above numerical value range.
(A1): A method of pulverizing particles with a ball mill or the like, dispersing the obtained coarse particles with a dispersing agent to obtain a desired particle size and then drying them.
(A2): A method of pulverizing particles with a ball mill or the like, and sieving the obtained coarse particles to screen a particle size.
(A3): A method of optimizing production conditions when a conductive material is produced, and adjusting the particle size of particles.

Here, the average particle size can be measured using a particle size distribution measuring device, an electron microscope or the like.

In the catalyst of the present invention, the content of the metal complex or adduct thereof with respect to a total amount of 100 mass% of the metal complex or adduct thereof and the conductive material is preferably 75 mass% or less, more preferably 50 mass% or less, and still more preferably 30 mass% or less. When the content of the metal complex or adduct thereof is equal to or less than the upper limit value, the conductivity of the catalyst is excellent. In addition, the content of the metal complex or adduct thereof with respect to a total amount of 100 mass% of the metal complex or adduct thereof and the conductive material is preferably 0.1 mass% or more, more preferably 0.5 mass% or more, and still more preferably 1 mass% or more. When the proportion of the metal complex or adduct thereof is the lower limit value or more, it is possible to further improve the oxygen reduction catalyst performance of the catalyst.

Although the application of the catalyst of the present invention is not particularly limited, it is preferably used for oxygen reduction because it has excellent oxygen reduction catalyst performance. Specifically, the catalyst of the present invention can be used for oxygen reduction electrodes in air batteries, fuel cells, electrochemical sensors and the like.

### [Liquid composition]

In one embodiment, the present invention relates to a liquid composition containing the catalyst of the present invention and a solvent. The solvent may be a solvent that easily dissolves the catalyst (that is, with high solubility) or a solvent that does not easily dissolve the catalyst (that is, with low solubility). When the solvent easily dissolves the catalyst, the liquid composition is in the form of a solution. When the solvent does not easily dissolve the catalyst, the liquid composition is in the form of a dispersion.

The solvent is not particularly limited, and may be an inorganic solvent such as water or an organic solvent. Specific examples of organic solvents include alcohols such as methanol, ethanol, propanol, isopropanol (2-propanol), and 1-hexanol; dimethylsulfoxide; tetrahydrofuran; aprotic polar solvents such as N-methylpyrrolidone, dimethylformamide, and acetone; and nonpolar solvents such as chloroform, dichloromethane, 1,4-dioxane, benzene, and toluene. The solvents may be used alone or two or more thereof may be used in combination.

The liquid composition can contain, as optional components, an optional conduction agent, a binder, and other additives. In addition, it may also contain a perfluorocarbon material containing a constituent unit based on polytetrafluoroethylene and a perfluoro side chain having a sulfonic acid group. Specific examples of perfluorocarbon materials include Nafion (product name: commercially available from Du Pont Inc.).

The liquid composition can be produced by mixing or kneading a catalyst, a solvent, and as necessary, a perfluorocarbon material. For mixing or kneading, an ultrasonic treatment, a mixer, a blender, a kneader, a homogenizer, a bead mill, a ball mill and the like may be used. Before and after the kneading operation, a sieve or the like may be used to adjust the average particle size of the particles. In addition, when a liquid composition containing a perfluorocarbon material is prepared, a catalyst, a perfluorocarbon material, and as necessary, water and an alcohol may be mixed and stirred until the mixture becomes uniform.

The liquid composition can be applied to the surface of various substrates. For example, a layer containing a catalyst (hereinafter referred to as a "catalyst layer") can be provided on the surface of various substrates by applying the liquid composition to the surface of the substrate and removing the solvent. That is, the liquid composition can be used, for example, as a coating solution to be applied to the surface of the substrate when an electrode is produced. The liquid composition may be directly used as a coating solution or may be used as a coating solution after the content of the catalyst or the solid content concentration is adjusted.

The substrate is not particularly limited, and examples thereof include aluminum alloys such as aluminum foils, electrolytic aluminum foils, aluminum meshes (expanded metals), foamed aluminum, perforated aluminum, and duralumin, copper alloys such as copper foils, electrolytic copper foils, copper meshes (expanded metals), foamed copper, perforated copper, and brass, brass foils, brass meshes (expanded metals), foam brass, perforated brass, nickel foils, nickel meshes, corrosion resistant nickel, nickel meshes (expanded metals), perforated nickel, foamed nickel, sponge nickel, zinc metal, corrosion resistant zinc metal, zinc foils, zinc meshes (expanded metals), steel plates, perforated steel plates, and silver. In addition, silicon base materials; metal base materials such as gold, iron, stainless steel, copper, aluminum, and lithium; alloy base materials containing any combination of these metals; oxide base materials such as indium tin oxide (ITO), indium zinc oxide (IZO), and antimony tin oxide (ATO); and base material-like substrates such as carbon base materials such as glassy carbon, pyrolytic graphite, and carbon felt can be used.

### [Electrode]

In one embodiment, the present invention relates to an electrode including the catalyst of the present invention. The electrode can have a layer containing the catalyst of the present invention (that is, catalyst layer) on the above substrate, which can be used as a catalyst for a reduction reaction, particularly, for an oxygen reduction reaction. The catalyst layer may be in direct contact with the substrate or another layer may be present between the substrate and the catalyst layer.

The method of producing an electrode is not particularly limited, and for example, it may be produced by applying a liquid composition to the surface of a conductive substrate and removing components other than the catalyst. When components other than the catalyst are removed, heating and drying may be performed, or pressing may be performed after drying. In addition, the catalyst layer may be provided on the surface of the substrate by vacuum deposition or the like. The electrode may have the catalyst layer only on one side of the substrate or may have the catalyst layer on both sides of the substrate.

The thickness of the catalyst layer is not particularly limited, and may be, for example, 0.01 to 100 µm. When the thickness is equal to or more than the lower limit value, the durability of the electrode is excellent. When the thickness is equal to or less than the upper limit value, the performance of the electrode is less likely to deteriorate.

The electrode can function as a catalyst for any electrochemical reaction such as a reduction reaction or an oxidation reaction, and for example, as a catalyst for reduction reactions shown below.

O₂+4H⁺+4e⁻→2H₂O

O₂+2H₂O+4e⁻→4OH⁻

### [Air battery]

In one embodiment, the present invention relates to an air battery including the electrode of the present invention. The electrode of the present invention can be used as an oxygen electrode of an air battery. The air battery can include an oxygen electrode, a metal electrode, an electrolyte, and a separator. In the present invention, the oxygen electrode is an electrode that uses gaseous oxygen as an electrode active material and is also called an air electrode.

The metal electrode is not particularly limited, and examples thereof include individual metals such as aluminum, magnesium, calcium, lithium, and zinc, and metal oxides thereof.

The electrolyte is preferably an aqueous electrolyte, and is not particularly limited, and examples thereof include alkaline aqueous solutions such as a potassium hydroxide aqueous solution and a sodium hydroxide aqueous solution and acidic aqueous solutions such as a sulfuric acid aqueous solution. The electrolytes may be used alone or two or more thereof may be used in combination. In addition, an inorganic solid electrolyte can be used.

The separator is a member that separates an oxygen electrode and a metal electrode, maintains an electrolyte, and secures ion conductivity between the oxygen electrode and the metal electrode. The separator is not particularly limited, and examples thereof include polymers having micropores such as polyethylene, polypropylene, polytetrafluoroethylene, polyvinylidene fluoride, cellulose, cellulose acetate, hydroxyalkylcellulose, carboxymethylcellulose, polyvinyl alcohol, cellophane, polystyrene, polyacrylonitrile, polyacrylamide, polyvinyl chloride, polyimide, polyamide, vinylon, and poly(meth)acrylic acid, gel compounds, ion exchange films, cyclized polymers, poly(meth)acrylate-containing polymers, sulfonate-containing polymers, quaternary ammonium salt-containing polymers, and quaternary phosphonium salt-containing polymers. The separator may be either a non-porous film or a porous film, and in the case of the porous film, the pore size is preferably 10 µm or less.

### [Fuel cell]

In one embodiment, the present invention relates to a fuel cell including the electrode of the present invention. The electrode of the present invention can be used as an oxygen electrode of a fuel cell. The fuel cell can include an oxygen electrode, a fuel electrode, an electrolyte, and a separator.

As the fuel electrode, the electrolyte, and the separator used in the fuel cell, those exemplified in the metal electrode, the electrolyte, and the separator used in the air battery can be used.

The fuel cell may be a primary battery or a secondary battery. Examples of forms of fuel cells include metal air batteries, molten carbonate fuel cells (MCFC), phosphoric acid fuel cells (PAFC), solid oxide fuel cells (SOFC), polymer electrolyte fuel cells (PEFC), enzymatic (bio) fuel cells, microbial fuel cells, hydrazine fuel cells, and direct-methanol fuel cells (DMFC). The forms of fuel cells are not limited to these examples, and PEFCs, enzymatic (bio) fuel cells, microbial fuel cells, hydrazine fuel cells or DMFCs are preferable. Examples of secondary batteries include hydrogen-air secondary batteries using a hydrogen storage alloy for a negative electrode.

### [Method of producing catalyst]

In one embodiment, the present invention relates to a method of producing the catalyst of the present invention. The method of producing the catalyst includes:
(a) a step of dissolving the metal complex or adduct thereof in a solvent to prepare a solution;
(b) a step of dispersing the conductive material in the solution to prepare a dispersion; and
(c) a step of removing the solvent from the dispersion.

The step (a) is a step of dissolving a metal complex or adduct thereof in a solvent to prepare a solution. The solution contains a metal complex or adduct thereof dissolved in a solvent and a solvent. Conditions such as the temperature and pressure when the solution is prepared are not particularly limited as long as the metal complex or adduct thereof can be dissolved in the solvent. For example, the temperature when the solution is prepared is preferably a temperature equal to or lower than a boiling point of the solvent, more preferably 5 to 80°C, and still more preferably 10 to 50°C. Most preferably, the solution is prepared at room temperature (25°C). In addition, regarding the pressure, for example, the solution can be prepared under atmospheric pressure.

The solvent is not particularly limited as long as a metal complex or adduct thereof can be dissolved in the solvent. The solubility of the metal complex or adduct thereof with respect to the solvent is preferably 0.1 g/L or more, more preferably 0.4 g/L or more, still more preferably 2.0 g/L or more, and particularly preferably 10 g/L or more. The upper limit value of the solubility of the metal complex or adduct thereof is not particularly limited, and may be, for example, 20 g/L or less, preferably 50 g/L or less, and more preferably 100 g/L or less. When the solubility of the metal complex or adduct thereof is the lower limit value or more, the metal complex or adduct thereof is easily dissolved in the solvent, and the metal complex or adduct thereof is more uniformly and easily adsorbed on the surface of the conductive material. As a result, oxygen reduction catalyst performance of the catalyst is improved, and durability when used as an electrode for an air battery or a fuel cell is further improved.

The solubility of the metal complex or adduct thereof with respect to the solvent is generally the maximum value of the dissolution amount (g) of the metal complex or adduct thereof per 1 L of the solvent measured using UV-visible spectroscopy at 25°C under atmospheric pressure. Here, the conditions for measuring the solubility of the metal complex or adduct thereof with respect to the solvent are unrelated to conditions for preparing the solution.

The step (b) is a step of dispersing a conductive material in a solution to prepare a dispersion, and in the step, the metal complex or adduct thereof can be adsorbed on the surface of the conductive material to form a complex. That is, the dispersion contains a complex in which a metal complex or adduct thereof is adsorbed on the surface of the conductive material. Conditions such as the temperature and pressure when the dispersion is prepared are not particularly limited as long as the conductive material can be dispersed. For example, the temperature when the dispersion is prepared is preferably a temperature equal to or lower than a boiling point of the solvent, more preferably 5 to 80°C, and still more preferably 10 to 50°C. Most preferably, the dispersion is prepared at room temperature (25°C).

The step (c) is a step of removing the solvent from the dispersion, and is a step of obtaining a complex in which a metal complex or adduct thereof is adsorbed on the surface of the conductive material as a catalyst. The method of removing the solvent from the dispersion is not particularly limited, and for example, it can be removed by solid-liquid separation. Filtration is preferable as a solid-liquid separation method because it reduces the temperature load on the catalyst. In filtration, the absorbance of the filtrate is preferably reduced by 10% or more compared to the solution. Thereby, it can be determined that the metal complex or adduct thereof is effectively adsorbed on the conductive material.

The method of producing the catalyst of the present invention is useful because it enables a complex of a metal complex or adduct thereof and a conductive material to be formed without performing a heat treatment at a high temperature. The method of producing the catalyst of the present invention can be performed at 200°C or lower, preferably 100°C or lower, and more preferably 50°C or lower. Since the metal complex or adduct thereof of the present invention has a relatively high solubility with respect to a solvent, it can be present in the solvent at a high concentration. In addition, since the metal complex or adduct thereof has high affinity with the conductive material, it can be effectively adsorbed on the surface of the conductive material. For example, the metal complex or adduct thereof can form a molecular layer of the metal complex or adduct thereof in a monomolecular state adsorbed on the surface of the conductive material.

In the method of producing the catalyst of the present invention, respective steps may be independent steps or a plurality of steps may be integrated. For example, the step (a) and the step (b) may be independent steps or may be performed simultaneously as the same step.

### [Examples]

While the present invention will be described below in more detail with reference to examples and comparative examples, the scope of the present invention is not limited to the examples.

### Synthesis Example 1 Synthesis of Compound (1)

410 mg of anhydrous ferric chloride and 5 mL of 1-pentanol were added to 1.29 g of pyridine-3,4-dicarbonitrile, 1.52 g of 1,8-diazabicyclo[5.4.0]undec-7-ene was added thereto, and the mixture was heated in an oil bath set to 160°C. After 3 hours, 20 mL of N,N-dimethylformamide was added thereto and the mixture was additionally heated for 1 hour. After cooling, 20 mL of methanol was added and the precipitated solid was collected by filtration. This solid was washed with methanol and dried to obtain Compound (1). The yield was 920 mg.

### Synthesis Example 2 Synthesis of Compound (2)

### Synthesis Example (2-1) Synthesis of diethyl pyridine-2,3-dicarboxylate N-oxide

150 mL of methylene chloride was added to 25.0 g of diethyl pyridine-2,3-dicarboxylate, and stirred. 100 mg of methyltrioxorhenium was added to this solution, and 3 mL of a 30% hydrogen peroxide solution was added thereto while checking the progress of the reaction by TLC. Stirring was continued for 2 days. Since residual raw materials were confirmed, 100 mg of methyltrioxorhenium and a hydrogen peroxide solution were added until the raw materials disappeared.

After the raw materials disappeared, 200 mL of water was added and stirred. The organic phase was subjected to liquid separation and the aqueous phase was then extracted with methylene chloride three times. The organic phase was combined and dehydrated with anhydrous sodium sulfate, then concentrated, and then purified through silica gel column chromatography.

A desired product was obtained as colorless crystals. The yield was 20.2 g.

### Synthesis Example (2-2) Synthesis of diethyl 6-chloropyrazine-2,3-dicarboxylate

15 mL of acetonitrile was added to 4.28 g of diethyl pyridine-2,3-dicarboxylate N-oxide, and stirred. 5.5 g of phosphorus oxychloride was added to this solution at room temperature, and the mixture was heated to reflux for 5 hours. After cooling, the reaction solution was poured into ice water, and extraction with ethyl acetate was performed. The organic phase was dried with anhydrous sodium sulfate and concentrated and then purified through silica gel column chromatography. The yield was 2.7 g.

### Synthesis (2-3) Synthesis of 6-methoxypyridine-2,3-dicarboxylic acid

20 mL of methanol was added to 2.5 g of diethyl 6-chloropyrazine-2,3-dicarboxylate, 2.0 g of potassium carbonate was additionally added and stirred at room temperature. After 4 hours, the inorganic substance was filtered off, and the solvent was distilled off under a reduced pressure. 20 mL of methanol was added to the residue again and stirred, and 1.63 g of lithium hydroxide/monohydrate was added thereto. After 5 hours, the reaction solution was poured into dilute aqueous hydrochloric acid, and extraction with ethyl acetate was performed.

The organic phase was dehydrated with anhydrous sodium sulfate and concentrated to obtain a desired product. The yield was 0.62 g.

### Synthesis Example (2-4) Synthesis of Compound (2)

500 mg of 6-methoxypyridine-2,3-dicarboxylic acid, 6 g of urea, 330 mg of ferric chloride hexahydrate, and 22 mg of hexaammonium heptamolybdate tetrahydrate were mixed and stirred, and heated in an oil bath set to 190°C. After the reaction for 3 hours, the mixture was cooled to 100°C, water was added thereto, and the mixture was additionally stirred for 10 minutes. After cooling to room temperature, the solid component was collected by filtration under a reduced pressure, washed with water, washed with methanol, and then finally washed with acetone and dried.

5 mL of N,N-dimethylformamide was added to the extracted solid component, and heated to reflux. After cooling, methanol was gradually added while stirring, the precipitated crystals were collected by filtration, washed with methanol, washed with acetone, and then dried to obtain Compound (2). The yield was 230 mg.

### Synthesis Example 3 Synthesis of Compound (3)

### Synthesis Example (3-1) Synthesis of dibutyl pyridine-3,4-dicarboxylate

100 mL of toluene, 40 mL of n-butanol, and 10 g of p-toluenesulfonic acid monohydrate were added to 18.0 g of pyridine-3,4-dicarboxylic acid and stirred. Using a soxhlet device and magnesium sulfate, the mixture was heated to reflux while removing the generated water.

After the reaction for 10 hours, the main products were separated using alumina column chromatography. The yield was 26.2 g.

### Synthesis Example (3-2) Synthesis of dibutyl pyridine-3,4-dicarboxylate N-oxide

100 mg of methyltrioxorhenium and 150 mL of methylene chloride were added to 25.0 g of dibutyl pyridine-3,4-dicarboxylate and stirred at room temperature. A 30% hydrogen peroxide solution was gradually added to this mixed solution. The progress of the reaction was followed by TLC. A hydrogen peroxide solution and methyltrioxorhenium were added and reacted for 2 days until the raw materials disappeared. After the reaction was completed, 100 mL of water was added thereto and liquid separation was performed.

The organic phase was washed with water and then additionally washed with a sodium sulfite aqueous solution. After dehydration with anhydrous sodium sulfate, concentration was performed to obtain a crude product as a reddish oil component. The yield was about 25 g.

### Synthesis Example (3-3) Synthesis of dibutyl 2-chloropyridine-4,5-dicarboxylate

10.0 g of dibutyl pyridine-3,4-dicarboxylate N-oxide was gradually added to 25 g of phosphorus oxychloride. After the mixture was stirred for 10 minutes, the reaction temperature was set to 110°C, and the reaction was performed for 5 hours. After cooling to room temperature, the mixture was carefully poured into a mixture containing 150 mL of ethyl acetate and 200 g of ice. After the mixture was stirred for 1 hour, liquid separation was performed, the organic phase was washed with a sodium bicarbonate aqueous solution, dehydrated with anhydrous sodium sulfate and then concentrated. There were two main products. These were separated and purified through silica gel column chromatography. Hexane and ethyl acetate were used as elution solvents, and desired dibutyl 2-chloropyridine-4,5-dicarboxylate was eluted first. The later eluted product was dibutyl 2-chloropyridine-3,4-dicarboxylate. The yields were all 7 g.

### Synthesis Example (3-4) Synthesis of dimethyl 2-methoxypyridine-4,5-dicarboxylate

2.5 g of dibutyl 2-chloropyridine-4,5-dicarboxylate was dissolved in 40 mL of methanol and stirred at room temperature. 2.0 g of potassium carbonate was added and stirred for 5 hours. The reaction solution was poured into dilute aqueous hydrochloric acid, extraction with ethyl acetate was performed, washing with water was performed, washing with a sodium bicarbonate aqueous solution was then performed, dehydration with anhydrous sodium sulfate was performed, and the residue was then crystallized with hexane. The yield was 0.9 g.

### Synthesis Example (3-5) Synthesis of 2-methoxypyridine-4,5-dicarboxylic acid

10 mL of methanol was added to 0.9 g of dimethyl 2-methoxypyridine-4,5-dicarboxylate and stirred at room temperature. 670 mg of lithium hydroxide monohydrate was added thereto and the mixture was reacted at room temperature. After 30 minutes, 670 mg of lithium hydroxide monohydrate was added and the reaction was additionally performed for one hour.

A homogeneous solution was obtained when acidified with concentrated hydrochloric acid. When water was added thereto and diluted, colorless crystals were precipitated. The crystals were collected by filtration, washed with water and then dried to obtain desired 2-methoxypyridine-4,5-dicarboxylic acid. The yield was 0.67 g.

### Synthesis Example (3-6) Synthesis of Compound (3)

250 mg of 2-methoxypyridine-4,5-dicarboxylic acid, 165 mg of ferric chloride hexahydrate, 11 mg of hexaammonium heptamolybdate tetrahydrate, and 3 g of urea were mixed and stirred, immersed in an oil bath at 185°C, and reacted. After the reaction for 1 hour, the temperature of the oil bath was set to 200°C, and the reaction was additionally performed for 2 hours. 5 mL of N-methylpyrrolidone was added thereto, the mixture was reacted for 1 hour, and then cooled to 80°C, methanol was added thereto, and the precipitated solid was collected by filtration.

This solid was adsorbed onto silica gel using N,N-dimethylformamide. The excess solvent was distilled off under a reduced pressure using a rotary evaporator. This adsorbent was placed on silica gel and eluted with N,N-dimethylformamide to obtain a purified desired product. The yield was 95 mg.

### Synthesis Example 4 Synthesis of Compound (6)

### Synthesis Example (4-1) Synthesis of 5,6-dimethoxy-2,3-pyrazinedicarbonitrile

15 mL of methanol was added to 2.0 g of 5,6-dichloro-2,3-pyrazinedicarbonitrile and stirred at room temperature. 3.0 g of potassium carbonate was added thereto and reacted overnight. 80 mL of water was added to the reaction solution, and the precipitated crystals were collected by filtration. Washing with water and washing with methanol were performed, and drying was performed to obtain a desired product. The yield was 1.90 g.

### Synthesis Example (4-2) Synthesis of Compound (6)

260 mg of anhydrous ferric chloride was added to 1.0 g of 5,6-dimethoxy-2,3-pyrazinedicarbonitrile, 5 mL of 1-pentanol and 1 mL of pyridine were additionally added thereto, the temperature of an oil bath was set to 155°C and the mixture was heated. After the reaction overnight, cooling was performed, methanol was added, and the precipitated solid was purified through silica gel column chromatography. A mixed solvent of chloroform and methanol was used as the elution solvent. The yield was 130 mg.

### Synthesis Example 5 Synthesis of Compound (7)

5 mL of 1-pentanol was added to 1.44 g of 5-methylpyrazine-2,3-dicarbonitrile, 410 mg of anhydrous ferric chloride and 1 mL of pyridine were added thereto, and the mixture was heated in an oil bath set to 150°C and reacted. After 7 hours, the mixture was cooled, 1 mL of concentrated hydrochloric acid and 2 mL of water were added thereto, 20 mL of methanol was additionally added thereto, and the precipitated solid was collected by filtration. The obtained solid was purified through silica gel column chromatography to obtain 80 mg of Compound (7).

### Synthesis Example 6 Synthesis of Compounds (8) and (9)

### Synthesis Example (6-1) Synthesis of 5,6-dimethyl-2,3-pyrazinedicarbonitrile

10 mL of acetic acid was added to 3.24 g of diaminomaleonitrile, 2.64 mL of diacetyl was added thereto and the mixture was heated to reflux for 1 hour. When radiative cooling was performed, crystals were precipitated. 10 mL of water was added thereto, and crystals were collected by filtration. After washing with water was performed, drying was performed to obtain a desired product. The yield was 4.45 g.

### Synthesis Example (6-2) Synthesis of Compound (9)

30 mL of 1-pentanol was added to 6.32 g of 5,6-dimethyl-2,3-pyrazinedicarbonitrile, 1.78 g of anhydrous ferric chloride, and 1.9 mL of 4-methylpyridine were additionally added thereto, and the mixture was heated in an oil bath set to 145°C and reacted. After the reaction for 5 hours, heating was stopped. When the temperature of the reaction solution reached 100°C, 30 mL of methanol was added dropwise. After radiative cooling to room temperature, filtration under a reduced pressure was performed, careful washing with water and methanol was performed, washing with acetone was then performed, and drying was performed. The yield was 6.8 g.

### Synthesis Example (6-3) Synthesis of Compound (8)

1.8 g of Compound (9) was taken, 20 mL of concentrated sulfuric acid was added and stirred at room temperature. After 1 hour, the mixture was added dropwise to 200 mL of water. The precipitated solid was extracted using a centrifuge, the obtained solid was washed with water and methanol using a centrifuge to obtain Compound (8). The yield was 1.0 g.

### Synthesis Example 7 Synthesis of Compound (10)

5 mL of 1-pentanol and 410 mg of anhydrous ferric chloride were added to 1.58 g of 5,6-dimethyl-2,3-pyrazinedicarbonitrile synthesized in Synthesis Example (6-1) described above and stirred. Then, 1.0 mL of 4-tert-butylpyridine was added thereto, and the mixture was heated in an oil bath set to 155°C. After 1 hour, 10 mL of N,N-dimethylformamide was added thereto, and the mixture was additionally stirred for 30 minutes. After cooling to room temperature, the precipitated solid was collected by filtration, washed with N,N-dimethylformamide and then washed with methanol to obtain a crude component of Compound (10). 15 mL of N,N-methylformamide was added to the obtained crude component and the mixture was heated to reflux for 30 minutes. After radiative cooling to room temperature, the solid was collected by filtration, and washed with N,N-dimethylformamide and then washed with methanol. This operation was repeated twice and drying was then performed to obtain Compound (10). The yield was 761 mg.

### Synthesis Example 8 Synthesis of Compound (11)

5 mL of 1-pentanol, 410 mg of anhydrous ferric chloride, and 1 mL of 4-methoxypyridine were mixed with 1.29 g of 2,3-dicyanopyrazine and stirred. This mixture was heated in an oil bath set to 155°C and reacted for 5 hours. After radiative cooling to room temperature, the solid was collected by filtration, washed with N,N-dimethylformamide and then washed with methanol, and dried to obtain a crude component of Compound (11). This crude component was purified through silica gel column chromatography to obtain Compound (11). The yield was 110 mg.

### Synthesis Example 9 Synthesis of Compound (13)

5 mL of 1-pentanol, 410 mg of anhydrous ferric chloride, and 1 mL of pyridine were mixed with 1.29 g of 2,3-dicyanopyrazine and stirred. This mixture was heated in an oil bath set to 150°C and reacted overnight. After radiative cooling to room temperature, 3 mL of water and 2 mL of concentrated hydrochloric acid were added, and 30 mL of methanol was added while stirring. The precipitated solid was collected by filtration and purified through silica column chromatography to obtain Compound (13). The yield was 405 mg.

### Synthesis Example 10 Synthesis of Compound (14)

410 mg of anhydrous ferric chloride and 5 mL of 1-pentanol were added to 1.3 g of 2,3-dicyanopyrazine and stirred. 1 mL of 4-tert-butylpyridine was added thereto and the mixture was heated in an oil bath set to 150°C. After the reaction for 5 hours, radiative cooling was performed to room temperature, and the precipitated solid was collected by filtration. Washing with methanol and washing with acetone were performed to obtain a crude component of Compound (14). 10 mL of N,N-dimethylformamide was added to the obtained crude Compound (14) and the mixture was heated to reflux for 1 hour. After radiative cooling to room temperature, the solid was collected by filtration, washed with methanol and washed with acetone to obtain Compound (14). The yield was 760 mg.

### Synthesis Example 11 Synthesis of Compound (15)

5 mL of 1-pentanol, 410 mg of anhydrous iron chloride, and 1 mL of 4-methoxypyridine were added to 1.29 g of 2,3-dicyanopyridine, and the mixture was heated in an oil bath set to 155°C. After the reaction for 5 hours, 10 mL of N,N-dimethylformamide was added thereto and heating was additionally continued for 30 minutes. After radiative cooling to room temperature, the precipitated solid was collected by filtration. This solid was washed with N,N-dimethylformamide, and then with methanol and acetone in that order, and dried to obtain Compound (15). The yield was 464 mg.

### Synthesis Example 12 Synthesis of Compound (17)

498 mg of ferrous chloride tetrahydrate, 2.4 g of urea, and 30 mg of hexaammonium heptamolybdate tetrahydrate were mixed with 1.67 g of pyridine-2,3-dicarboxylic acid and the mixture was reacted in an oil bath set to 180°C for 1 hour. After radiative cooling to room temperature, water was added, the solid was loosened and collected by filtration under a reduced pressure, washed with water, washed with methanol, and washed with acetone to obtain 650 mg of a dark green solid. Then, 7 mL of dimethylsulfoxide was added to this solid, 5 g of methyl p-toluenesulfonate was added, and the reaction was performed at an internal temperature of 75°C for 22 hours. Cooling was performed to room temperature and acetone was added until the solid was precipitated. The solid was collected by filtration and then dissolved in methanol.

A separate methanol solution prepared from 3 g of ammonium hexafluorophosphate was added to this methanol solution. The solid was precipitated and collected by filtration. The obtained solid was washed with methanol and then washed with acetone to obtain about 150 mg of a solid.

30 mL of methanol was added to this solid and the mixture was heated to reflux for 15 minutes, and after radiative cooling to room temperature, the solid was collected by filtration. Washing with methanol was performed and drying was performed to obtain Compound (17). The yield was 110 mg.

### Synthesis Example 13 Synthesis of Compound (18)

1.95 g of 5-ethylpyridine-2,3-dicarboxylic acid, 10 g of urea, 87 mg of hexaammonium heptamolybdate tetrahydrate, and 1.35 g of ferric chloride tetrahydrate were mixed, and the mixture was heated in an oil bath set to 190°C and reacted for 3 hours. Cooling was performed, water was added, and the solid was loosened and collected by filtration. 120 mL of water was added to this solid and the mixture was heated to reflux for 30 minutes. After cooling, this solid was collected by filtration and purified through silica gel column chromatography and then dissolved in concentrated sulfuric acid. This concentrated sulfuric acid solution was added dropwise to 500 mL of water, and the precipitated solid was collected using a centrifuge. This solid was washed with methanol and dried to obtain a desired product. The yield was 822 mg.

### Synthesis Example 14 Synthesis of Compound (19)

10 mL of 1-pentanol was added to 1.29 g of 2,3-dicyanopyridine and 390 mg of anhydrous cobalt chloride, 0.1 mL of 1,8-diazabicyclo[5.4.0]undec-7-ene was added thereto and the mixture was then heated to 155°C and reacted overnight.

20 mL of N,N-dimethylformamide was added to this reaction solution, and the mixture was additionally stirred at 155°C for 30 minutes. Cooling was performed to room temperature, the precipitated solid was collected by filtration under a reduced pressure, washed with N,N-dimethylformamide, additionally washed with water and methanol, and then washed with acetone and dried to obtain a desired product. The yield was 0.75 g.

### Synthesis Example 15 Synthesis of Compound (20)

### Synthesis Example (15-1) Synthesis of thiophene-2,3-dicarbaldehyde dioxime

5.2 g of thiophene-2,3-dicarbaldehyde, 50 mL of methanol, and 6.3 g of hydroxylamine hydrochloride were mixed and stirred, 7.4 g of sodium acetate was added thereto and the mixture was heated to reflux for 6 hours. 0.9 g of hydroxylamine hydrochloride was added thereto and the mixture was then reacted overnight.

After concentration, ethyl acetate and water were added and extraction was performed. The organic phase was dehydrated with anhydrous sodium sulfate and then concentrated, and hexane and ethyl acetate were added, and the mixture was crystallized. This was collected by filtration to obtain thiophene-2,3-dicarbonitrile dioxime. The yield was about 6 g.

### Synthesis Example (15-2) Synthesis of thiophene-2,3-dicarbonitrile

About 6 g of the dioxime was dissolved in 60 mL of acetonitrile, 16 mL of trimethylamine and 11.5 g of acetic anhydride were added thereto and the mixture was reacted at room temperature for 5 days. After concentration, water and ethyl acetate were added and extraction was performed, the organic phase was dried with anhydrous sodium sulfate and then purified through silica gel column chromatography to obtain thiophene-2,3-dicarbonitrile. The yield was 3.8 g.

### Synthesis Example (15-3) Synthesis of Compound (20)

1 g of a methanol solution containing 10% lithium methoxide was added to 3 mL of 1-octanol, the mixture was heated in an oil bath set to 230°C, and methanol was evaporated. 360 mg of thiophene-2,3-dicarbonitrile was added to this solution. After the reaction for 15 minutes, cooling was performed to 100°C, and 272 mg of ferric chloride hexahydrate was added. The reaction was performed for 1 hour, 15 mL of methanol was added, and the precipitated solid was collected by filtration. N,N-dimethylformamide and silica gel were added to the obtained solid, heated and dissolved and the solvent was then distilled off under a reduced pressure. This solid was placed on a silica gel column and eluted with N,N-dimethylformamide, and a cyan component was collected and concentrated to obtain Compound (20). The yield was 30 mg.

### Synthesis Example 16 Synthesis of Compounds (21) and (22)

### Synthesis Example (16-1) Synthesis of dimethyl 5-bromopyrazine-2,3-dicarboxylate

250 mL of methanol was added to 30.0 g of pyridine-2,3-dicarboxylic acid and stirred. 10 mL of concentrated sulfuric acid was added dropwise to this reaction solution, and 20 mL of trimethyl orthoformate was added dropwise. The mixture was heated to reflux for 2 days, 20 mL of trimethyl orthoformate was added thereto, and the mixture was additionally refluxed for 1 day. After cooling to 40°C, the temperature was maintained at 55°C or lower, and 16 mL of bromine was gradually added while checking the progress of the reaction by TLC. After 1 day, 8 mL of bromine was added and the reaction was additionally performed at 58°C for 3 days. This reaction solution was gradually poured while stirring ethyl acetate, water, and sodium hydrogen carbonate (in amounts capable of neutralizing the acid component). After liquid separation, the organic phase was washed with a sodium sulfite aqueous solution. The organic phase was concentrated after water was removed with anhydrous sodium sulfate, and cooled and isopropanol was added to crystallize and filtration was performed. After washing with isopropanol, washing with hexane was performed and drying was performed to obtain a desired product. The yield was 34.4 g.

### Synthesis Example (16-2) Synthesis of dimethyl 5-(2-ethylhexylthio)pyridine-2,3-dicarboxylate

5.9 g of dimethyl 5-bromopyrazine-2,3-dicarboxylate was added to 3.2 g of 2-ethylhexanethiol, 25 mL of N,N-dimethylformamide was additionally added and stirred. 6.1 g of potassium carbonate was added thereto, the temperature of the oil bath was then set to 85°C and heating was performed. The reaction was performed overnight, and 120 mL of ethyl acetate was added. This organic phase was washed with a potassium carbonate aqueous solution and then concentrated and purified through silica gel column chromatography to obtain a desired product. The yield was 6.2 g.

### Synthesis Example (16-3) Synthesis of 5-(2-ethylhexylthio)pyridine-2,3-dicarboxylic acid

50 mL of methanol was added to 5.0 g of dimethyl 5-(2-ethylhexylthio)pyridine-2,3-dicarboxylate, and 2.5 g of lithium hydroxide monohydrate was added thereto. After the reaction was completed, acidifying with dilute hydrochloric acid was performed and extraction with ethyl acetate was performed. The organic phase was washed with water three times and concentrated. The operation of adding toluene to this concentrate and performing distilling off under a reduced pressure was repeated three times to obtain a desired product.

### Synthesis Example (16-4) Synthesis of Compound (21)

3.2 g of urea and 30 mg of hexaammonium heptamolybdate tetrahydrate were added to 1.07 g of 5-(2-ethylhexylthio)pyridine-2,3-dicarboxylic acid, and the mixture was heated in an oil bath set to 160°C for 30 minutes. 465 mg of ferric chloride hexahydrate and 3.2 g of urea were added to the reaction mixture, the oil bath was set to 200°C, and the mixture was reacted for 1 hour. 6 mL of N-methylpyrrolidone was added, and heating was additionally performed for 1 hour. After cooling to room temperature, water was added, the precipitated solid was collected by filtration, washed with methanol, and then purified through silica gel column chromatography to obtain Compound (21). The yield was 320 mg.

### Synthesis Example (16-5) Synthesis of dimethyl 5-(2-ethylhexylsulfonyl)pyridine-2,3-dicarboxylate

80 mL of acetic acid and 300 mg of sodium tungstate dehydrate were added to 6.0 g of dimethyl 5-(2-ethylhexylthio)pyridine-2,3-dicarboxylate and stirred. This was heated to 40°C, and 4.4 mL of a 30% hydrogen peroxide solution was gradually added dropwise while checking the progress of the reaction by TLC. After a total amount of the hydrogen peroxide solution was added dropwise, the reaction was performed for 1 hour, and 150 mL of water was added. After extraction with ethyl acetate was performed, the organic phase was washed with a sodium bicarbonate aqueous solution twice and washed with a sodium sulfite aqueous solution twice, and water was then removed with anhydrous sodium sulfite, and concentration was then performed. The residue was purified through silica gel column chromatography to obtain a desired product as a colorless oil component. The yield was 6.0 g.

### Synthesis Example (16-6) Synthesis of 5-(2-ethylhexylsulfonyl)pyridine-2,3-dicarboxylic acid

150 mL of methanol was added to 13.6 g of dimethyl 5-(2-ethylhexylsulfonyl)pyridine-2,3-dicarboxylate and stirred. 6.8 g of lithium hydroxide was added thereto and the mixture was reacted at room temperature for 2 hours. After the reaction was completed, acidifying with dilute hydrochloric acid was performed and extraction with ethyl acetate was performed. The organic phase was washed with water three times and concentrated. The operation of adding toluene to this concentrate and performing distilling off under a reduced pressure was repeated three times to obtain a desired product.

### Synthesis Example (16-7) Synthesis of Compound (22)

3.0 g of urea and 25.3 mg of hexaammonium heptamolybdate tetrahydrate were added to 1.0 g of 5-(2-ethylhexylsulfonyl)pyridine-2,3-dicarboxylic acid, and the mixture was heated in an oil bath set to 160°C for 30 minutes. Then, 400 mg of ferric chloride hexahydrate and 3.0 g of urea were added, the oil bath was set to 200°C, and the mixture was reacted for 3 hours. After cooling to room temperature, water was added, and the precipitated solid was collected by filtration. This solid was purified through silica gel column chromatography to obtain Compound (22). The yield was 199 mg.

### Synthesis Example 17 Synthesis of Compound (23)

### Synthesis Example (17-1) Synthesis of dimethyl 4-bromophthalate

100 mL of methanol was added to 10 g of 4-bromophthalic anhydride and the mixture was refluxed for 1 hour. After cooling to room temperature, 5 g of trimethyl orthoformate was added and 10 mL of acetyl chloride was slowly added dropwise. This reaction solution was heated and refluxed for 3 days. After concentration, ethyl acetate and a sodium hydrogen carbonate solution were added, liquid separation was performed, and the organic phase was then concentrated after water was removed with anhydrous sodium sulfate to obtain a desired product. The yield was 10.8 g.

### Synthesis Example (17-2) Synthesis of dimethyl 4-(2-ethylhexylthio)phthalate

8.18 g of dimethyl 4-bromophthalate, 6.3 g of potassium carbonate and 40 mL of N,N-dimethylformamide were added to 4.38 g of 2-ethylhexanethiol, and the inside of the system was purged with nitrogen. This reaction solution was heated to 100°C and reacted overnight. After cooling, ethyl acetate and water were added, extraction was performed, and concentration was performed, and purification was then performed through silica gel column chromatography to obtain a desired product. The yield was 5.2 g.

### Synthesis Example (17-3) Synthesis of dimethyl 4-(2-ethylhexylsulfonyl)phthalate

50 mL of acetic acid and 250 mg of sodium tungstate dehydrate were added to 5.0 g of dimethyl 4-(2-ethylhexylthio)phthalate and stirred. Heating was performed in an oil bath set to 40°C and 3.7 mL of a 30% hydrogen peroxide solution was gradually added dropwise. After the reaction for 1 hour, the oil bath was set to 75°C, and the reaction was performed for 4 hours. After the reaction was completed, the temperature was set to room temperature, water and ethyl acetate were added, and extraction was performed. The organic phase was washed with a sodium bicarbonate aqueous solution, and additionally washed with a sodium sulfite solution. Sodium sulfite was added, water was removed, and the organic phase was concentrated. The residue was purified through silica gel column chromatography to obtain a desired product. The yield was 4.78 g.

### Synthesis Example (17-4) Synthesis of 4-(2-ethylhexylsulfonyl)phthalate

30 mL of methanol was added to 4.4 g of dimethyl 4-(2-ethylhexylsulfonyl)phthalate, 2.2 g of lithium hydroxide monohydrate was added thereto and the mixture was reacted room temperature overnight. Acidifying with concentrated hydrochloric acid was performed, and concentration was performed. Ethyl acetate and water were added, extraction was performed, the organic phase was washed with a saturated saline solution, water was then removed with anhydrous sodium sulfate and concentration was performed. A desired product was solidified from an oil component and obtained as a colorless solid. The yield was 3.8 g.

### Synthesis Example (17-5) Synthesis of Compound (23)

4.5 g of urea and 40 mg of hexaammonium heptamolybdate tetrahydrate were added to 1.5 g of 5-(2-ethylhexylsulfonyl)phthalate, and the mixture was heated in an oil bath set to 165°C for 30 minutes. Then, 600 mg of ferric chloride hexahydrate and 4.5 g of urea were added, the oil bath was set to 200°C, and the mixture was reacted for 1 hour. After cooling, water was added, and the precipitated solid was collected by filtration and purified through silica gel column chromatography to obtain Compound (23). The yield was 250 mg.

### Synthesis Example 18 Synthesis of Compound (24)

### Synthesis Example (18-1) Synthesis of diethyl 2-phenylpyrimidine-4,5-dicarboxylate

Water was added to dissolve 21.0 g of diethyl oxaloacetate sodium salt, 10 mL of concentrated hydrochloric acid was added to acidify it, extraction was performed with ethyl acetate, and concentration was performed. 22.2 g of triethyl orthoformate and 31.0 g of acetic anhydride were added to the residue, and the mixture was heated at 120°C for 30 minutes, 140°C for 1 hour, and 150°C for 2 hours while trapping the distillate component. Then, the pressure was reduced while heating to 80°C, and the component to be distilled off was removed. The operation of adding toluene to the residue and removing the azeotropic component was repeated.

Separately, 50 mL of ethanol was added to 6.24 g of benzamidine hydrochloride and stirred, and a 20% sodium ethoxide ethanol solution was added until it became basic. The above toluene azeotropic residue was added to this reaction solution. After stirring was performed at room temperature for 30 minutes, the reaction was performed at 85°C for 3 hours. After cooling, ethyl acetate and water were added, extraction was performed, and the organic phase was concentrated and purified through silica gel column chromatography. A desired product was precipitated as crystals from hexane. The yield was 1.0 g.

### Synthesis Example (18-2) Synthesis of 2-phenylpyrimidine-4,5-dicarboxylic acid

30 mL of methanol was added to 1.7 g of diethyl 2-phenylpyrimidine-4,5-dicarboxylate, and 1.0 g of lithium hydroxide monohydrate was added. The mixture was reacted at room temperature overnight and then concentrated, and water was added. When acidifying with concentrated hydrochloric acid was performed, crystals were precipitated. These were collected by filtration and washed with cold water and then dried to obtain a desired product. The yield was 1.36 g.

### Synthesis Example (18-3) Synthesis of Compound (24)

5 g of urea, 22 mg of hexaammonium heptamolybdate tetrahydrate, and 330 mg of ferric chloride hexahydrate were added to 600 mg of 2-phenylpyrimidine-4,5-dicarboxylic acid, and the mixture was heated in an oil bath set to 185°C for 2 hours. Next, the temperature of the oil bath was set to 205°C, and the reaction was performed for 2 hours. Then, 3 mL of N-methylpyrrolidone was added and the mixture was heated for 4 hours while the temperature was set to 205°C. After cooling to room temperature, 40 mL of methanol and 40 mL of water were added, and the precipitated solid was collected by filtration.

This solid was purified through silica gel column chromatography. The impurities were eluted with a mixed solution containing chloroform and methanol and a desired product was then eluted with N,N-dimethylformamide. The solvent was distilled off, methanol was added, collection by filtration was performed, and drying was performed to obtain Compound (24). The yield was 198 mg.

### <Example 1>

Compound (1): 0.1 mg was dissolved in 1.0 mL of dimethylsulfoxide (DMSO) to prepare a solution in which the concentration of Compound (1) was 0.1 g/L. 5 mg of carbon black having a carboxyl group was dispersed in the obtained solution. For dispersion, an ultrasonic treatment (20 kHz) was performed for 15 minutes. The solvent DMSO was removed from the obtained dispersion through solid-liquid separation and washing with methanol, and drying was performed at room temperature for 24 hours to obtain a catalyst of Example 1.

Next, 0.82 mg of the obtained catalyst of Example 1, 84 µL of Milli-Q water, 336 µL of isopropyl alcohol, and 6 µL of a 0.5 mass% Nafion aqueous solution were kneaded with an ultrasonic stirrer and applied to a glassy carbon (GC) electrode to obtain an electrode of Example 1.

### (Half-wave potential)

In the Linear Sweep Voltammetry (LSV) curve, the potential at which the current value reached half of the current value when the potential was 0.5 (V vs. RHE) was defined as the half-wave potential.

### (LSV curve)

The LSV curve was obtained using an oxygen-saturated 0.1 M potassium hydroxide aqueous solution as an electrolytic solution and a rotation ring disk electrode (RRDE-3A, commercially available from BAS Inc.) under conditions of a sweep rate of 5 mV/s. The rotation speed of the rotation disk was 1,600 rpm, and a Pt line was used as a counter electrode, and Ag/AgCl was used as a reference electrode.

### (LSV measurement by RRDE)

LSV measurement by RRDE was performed using a rotation ring disk electrode (RRDE-3A, commercially available from BAS Inc.) and an oxygen-saturated 0.1 M potassium hydroxide aqueous solution as an electrolytic solution under conditions of a sweep rate of 5 mV/s. Regarding the rotation speed of the rotation disk, the LSV was measured at each of the rotation speeds of 0 rpm, 400 rpm, 800 rpm, 1,200 rpm, 1,600 rpm, 2,000 rpm, and 2,400 rpm. Pt was used as a counter electrode, and Ag/AgCl was used as a reference electrode.

FIG. 1 shows examples of the results of LSV measurement by RRDE. In the graph of FIG. 1, a higher applied potential (starting potential) shown on the horizontal axis when current generation starts on the vertical axis indicates better oxygen reduction catalyst performance.

The starting potential and the half-wave potential were measured under the same conditions as in Example 1 except that metal complexes shown in the following Table 1 were used in place of the metal complex (Compound (1)) in Example 1. In addition, in Comparative Example 1, manganese dioxide was used in place of the metal complex. The results are shown in Table 1.

### [Table 1]

**Table 1 Starting potential and half-wave potential of examples and comparative example**

| | Example 1 | Example 2 | Example 3 | Example 4 | Example 5 | Comparativ e Example 1 |
|---|---|---|---|---|---|---|
| | Compoun d (1) | Compoun d (4) | Compoun d (8) | Compoun d (21) | Compoun d (22) | Manganese dioxide |
| Starting potentia 1 [V vs. RHE] | 1.000 | 1.010 | 1.010 | 0.995 | 1.000 | 0.890 |
| Half-wave potentia 1 [V vs. RHE] | 0.906 | 0.922 | 0.928 | 0.923 | 0.919 | 0.804 |

As shown in Table 1, it was found that the catalysts of Examples 1 to 5 according to the present invention had a higher starting potential and half-wave potential and better oxygen reduction catalyst performance than the catalyst of Comparative Example 1.

### [Industrial Applicability]

The metal complex or adduct thereof of the present invention is useful because it has excellent oxygen reduction catalyst performance when used as a catalyst. In addition, since it can be easily adsorbed to the conductive material and does not use rare metals, the production cost can be reduced, and a production process suitable for mass production can be designed.

## Claims

1. A metal complex or adduct thereof, which is represented by the following Formula (1) or (2): 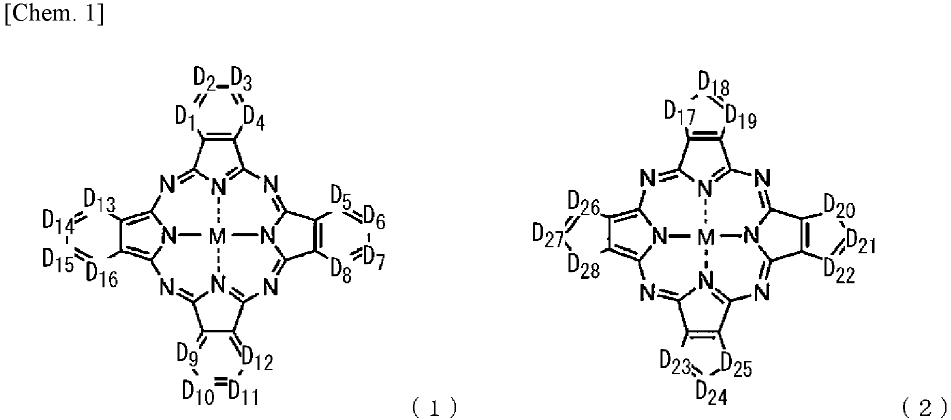 (where,
M represents an iron atom or a cobalt atom,
D¹ to D²⁸ each independently represent a nitrogen atom, a sulfur atom or a carbon atom,
and when D¹ to D²⁸ are carbon atoms, the carbon atoms may be each independently bonded to a hydrogen atom, a halogen atom, an alkyl group, a cycloalkyl group, an alkenyl group, an alkynyl group, an aryl group, an alkylsulfonyl group, an alkoxy group or an alkylthio group)
where, in Formula (1), when the number of carbon atoms among D¹ to D¹⁶ is 8 or less,
at least one of the carbon atoms is bonded to a halogen atom, an alkyl group, a cycloalkyl group, an alkenyl group, an alkynyl group, an aryl group, an alkylsulfonyl group, an alkoxy group or an alkylthio group or
the metal complex of Formula (1) forms an adduct.

2. The metal complex or adduct thereof according to claim 1, wherein D¹ to D¹⁶ represent a nitrogen atom or a carbon atom.

3. The metal complex or adduct thereof according to claim 1 or 2,
wherein D¹⁷ to D²⁸ represent a sulfur atom or a carbon atom.

4. The metal complex or adduct thereof according to any one of claims 1 to 3, which is represented by the following Formulae:

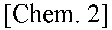

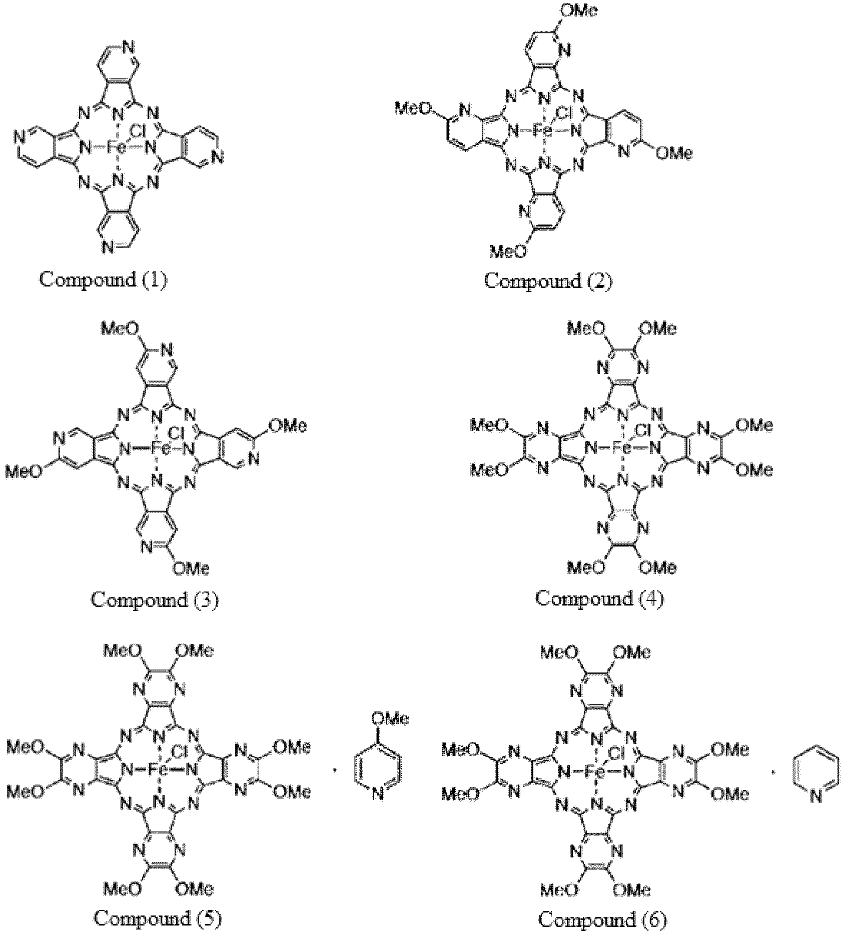

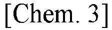

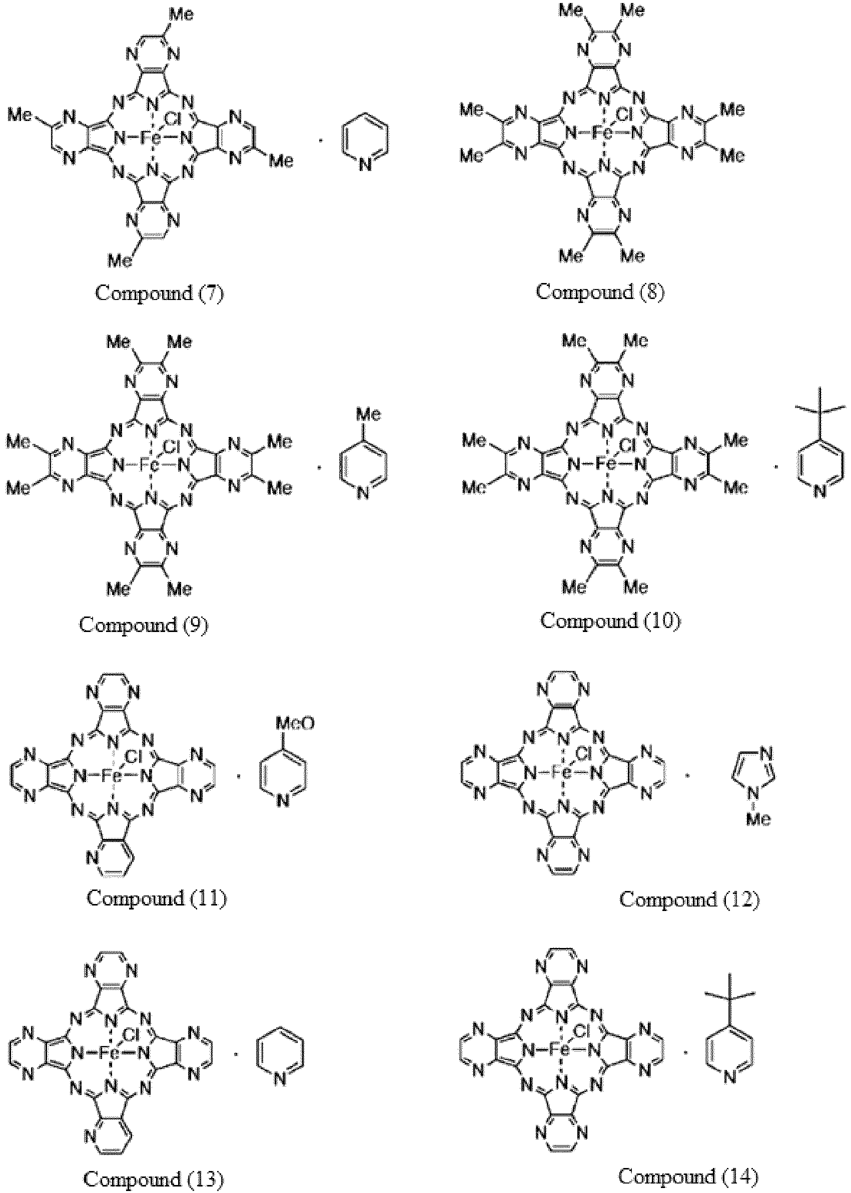

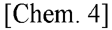

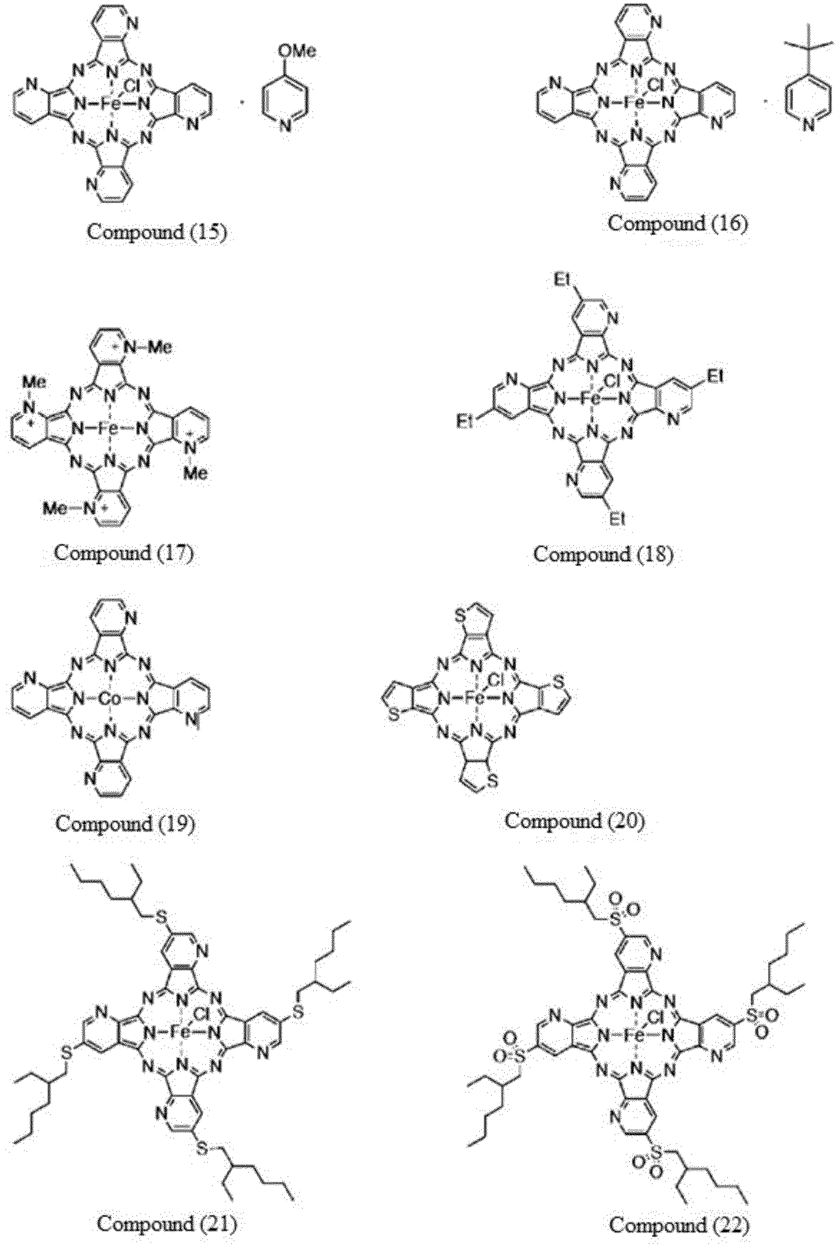

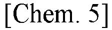

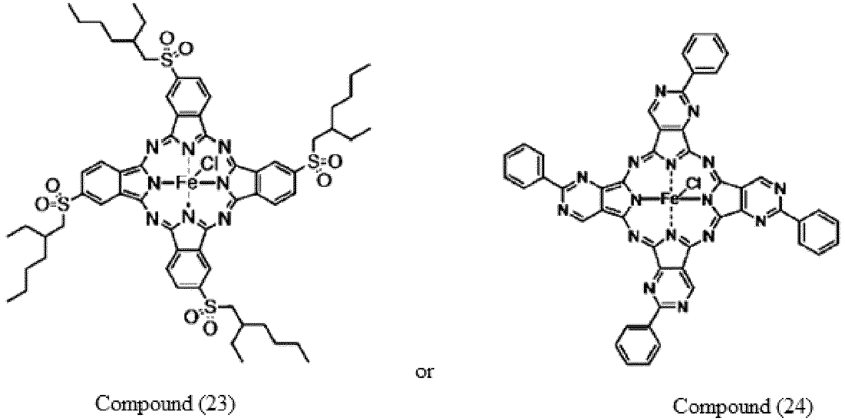

5. A catalyst comprising the metal complex or adduct thereof according to any one of claims 1 to 4 and a conductive material.

6. The catalyst according to claim 5,
wherein the content of the metal complex is 75 mass% or less with respect to a total amount of 100 mass% of the metal complex or adduct thereof and the conductive material.

7. The catalyst according to claim 5 or 6,
wherein the conductive material contains a carboxyl group.

8. The catalyst according to claim 7,
wherein the content of the carboxyl group is 20 mass% or less with respect to 100 mass% of the conductive material.

9. The catalyst according to any one of claims 5 to 8, which is for oxygen reduction.

10. A liquid composition comprising the catalyst according to any one of claims 5 to 8 and a solvent.

11. An electrode comprising the catalyst according to any one of claims 5 to 8.

12. An air battery or fuel cell comprising the electrode according to claim 11.

13. A method of producing the catalyst according to any one of claims 5 to 8, comprising:
(a) a step of dissolving the metal complex or adduct thereof in a solvent to prepare a solution;
(b) a step of dispersing the conductive material in the solution to prepare a dispersion; and
(c) a step of removing the solvent from the dispersion.

14. The production method according to claim 13,
wherein the steps (a) and (b) are performed at a temperature equal to or lower than a boiling point of the solvent.

15. The production method according to claim 13 or 14,
wherein the solubility of the metal complex or adduct thereof with respect to the solvent is 0.1 g/L or more.
